# EUROPÄISCHE PATENTSCHRIFT

(11) **EP 2 683 681 B1**
(45) Veröffentlichungstag und Bekanntmachung des Hinweises auf die Patenterteilung: **01.07.2015**
(21) Anmeldenummer: 12708115.6
(22) Anmeldetag: 01.03.2012
(51) Int. Cl.: B01J 31/18, B01J 31/22, C07C 209/16, C07C 213/02, C07D 307/52

(54) **VERFAHREN ZUR HERSTELLUNG VON DI-, TRI- UND POLYAMINEN DURCH HOMOGEN-KATALYSIERTE ALKOHOLAMINIERUNG**
METHOD FOR PRODUCING DIAMINES, TRIAMINES AND POLYAMINES BY HOMOGENEOUSLY CATALYZED ALCOHOL AMINATION
PROCÉDÉ DE PRODUCTION DE DIAMINES, DE TRIAMINES ET DE POLYAMINES PAR AMINATION D'ALCOOLS À L'AIDE D'UN CATALYSEUR HOMOGÈNE

(30) Priorität: 08.03.2011 EP 11157342
(43) Veröffentlichungstag der Anmeldung: 15.01.2014
(73) Patentinhaber: BASF SE, 67056 Ludwigshafen (DE)
(72) Erfinder: SCHAUB, Thomas, 67433 Neustadt (DE); BUSCHHAUS, Boris, 68161 Mannheim (DE); BRINKS, Marion Kristina, 68165 Mannheim (DE); SCHELWIES, Mathias, 69115 Heidelberg (DE); PACIELLO, Rocco, 67098 Bad Dürkheim (DE); MELDER, Johann-Peter, 67459 Böhl-Iggelheim (DE); MERGER, Martin, 67227 Frankenthal (DE)
(86) Internationale Anmeldenummer: PCT/EP2012/053584
(87) Internationale Veröffentlichungsnummer: WO 2012/119929

(56) Entgegenhaltungen:
- WO-A1-2010/018570
- SEBASTIAN IMM ET AL: "An Efficient and General Synthesis of Primary Amines by Ruthenium-Catalyzed Amination of Secondary Alcohols with Ammonia", ANGEWANDTE CHEMIE. INTERNATIONAL EDITION, WILEY VCH VERLAG, WEINHEIM, Bd. 49, Nr. 44, 25. Oktober 2010 (2010-10-25), Seiten 8126-8129, XP002669003, ISSN: 1433-7851, DOI: 10.1002/ANIE.201002576 [gefunden am 2010-08-02]

## Beschreibung

Die vorliegende Erfindung betrifft ein Verfahren zur Herstellung von primären Di-, Tri- und Polyaminen durch homogen-katalysierte Alkoholaminierung von Di-, Tri- und Polyolen sowie von Alkanolaminen, die mindestens eine primäre Hydroxylgruppe aufweisen, mit Ammoniak unter Wasserabspaltung in Gegenwart eines Komplexkatalysators, der mindestens ein Element ausgewählt aus den Gruppen 8, 9 und 10 des Periodensystems sowie mindestens einen Donorliganden enthält.

Primäre Amine sind Verbindungen, die mindestens eine primäre Aminogruppe (-NH₂) aufweisen. Primäre Diamine weisen zwei primäre Aminogruppen auf. Primäre Triamine weisen drei primäre Aminogruppen auf. Primäre Polyamine weisen mehr als drei primäre Aminogruppe auf.

Primäre Amine sind wertvolle Produkte mit einer Vielzahl unterschiedlicher Verwendungen, beispielsweise als Lösungsmittel, Stabilisatoren, zur Synthese von ChelatBildnern, als Edukte zur Herstellung von Kunstharzen, Inhibitoren, grenzflächenaktiven Substanzen, Zwischenprodukte bei der Herstellung von Kraftstoffadditiven (US 3,275,554 A, DE 2125039 A und DE 36 11 230 A), Tensiden, Arznei- und Pflanzenschutzmitteln, Härtern für Epoxyharze, Katalysatoren für Polyurethane, Zwischenprodukte zur Herstellung quaternärer Ammoniumverbindungen, Weichmachern, Korrosionsinhibitoren, Kunstharzen, Ionenaustauschern, Textilhilfsmitteln, Farbstoffen, Vulkanisationsbeschleunigern und/oder Emulgatoren.

WO 2010/018570 beschreibt einen Prozess zur Herstellung von Aminen.

Primäre Di- und Triamine werden gegenwärtig durch heterogen-katalysierte Alkoholaminierung von primären Di- und Triolen mit Ammoniak hergestellt. Die WO 2008/006752 A1 beschreibt ein Verfahren zur Herstellung von Aminen durch Umsetzung von primären oder sekundären Alkoholen mit Ammoniak in Gegenwart eines heterogenen Katalysators, der Zirkoniumdioxid und Nickel enthält. Die WO 03/051508 A1 betrifft ein Verfahren zur Aminierung von Alkoholen unter Verwendung von spezifischen heterogenen Cu/Ni/Zr/Sn-Katalysatoren. Aus der EP 0 696 572 A1 sind Nickel-, Kupfer-, Zirkonium- und Molybdänoxid enthaltende heterogene Katalysatoren zur Aminierung von Alkoholen mit Ammoniak und Wasserstoff bekannt. Die Reaktionen werden in den vorstehend genannten Dokumenten bei Temperaturen im Bereich von 150 bis 210 °C und Ammoniakdrücken im Bereich von 30 bis 200 bar durchgeführt. Bei den in den vorstehenden Dokumenten beschriebenen heterogen-katalysierten Verfahren entstehen als Hauptprodukte jedoch die unerwünschten Monoaminierungsprodukte und zyklische Amine wie Piperazine, Pyrrolidine und Morpholine. Die gewünschten primären Diamine werden bei den vorstehend beschriebenen Verfahren nicht oder lediglich in äußerst geringen Ausbeuten erhalten. In den vorstehend genannten Dokumenten ist insbesondere die Umsetzung von Diethylenglykol mit Ammoniak beschrieben.

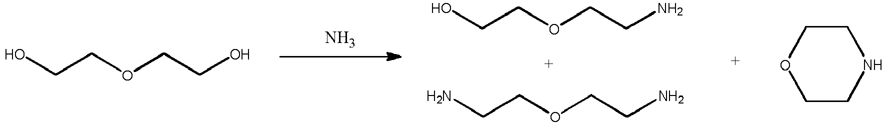

Hierbei werden als Hauptprodukte Monoaminodiethylenglykol und Morpholin erhalten. Das gewünschte zweifach aminierte Diaminodiethylenglykol wird bei den Aminierungsreaktionen der vorstehend genannten Dokumente nicht oder nur in äußerst geringen Ausbeuten erhalten.

Die höchste Ausbeute an Diaminodiethylenglykol mit 5 % wird gemäß WO 03/051508 A1 erhalten, wobei als Nebenprodukte 22% Morpholin und 36 % Monoaminodiethylenglykol entstehen.

Bei der Aminierung von Diethanolamin mit Ammoniak wird als Hauptprodukt Piperazin erhalten. Auch hier fallen das Monoaminierungsprodukt und das gewünschte lineare Diaminierungsprodukt Diethylentriamin nur in Spuren an.

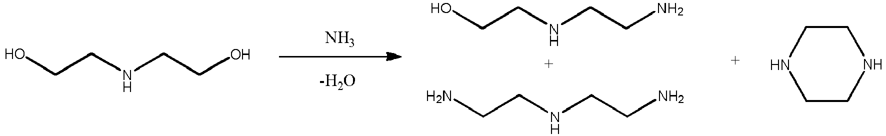

Bei der Umsetzung von Polyetherolen werden mit den vorstehend beschriebenen Verfahren für die heterogen-katalysierte Aminierung in hohem Ausmaß unerwünschte Nebenreaktionen zum dimeren sekundären Amin oder polymeren Kopplungsprodukt beobachtet. Diese Nebenprodukte sind schwer vom gewünschten primären Diaminierungsprodukt abtrennbar.

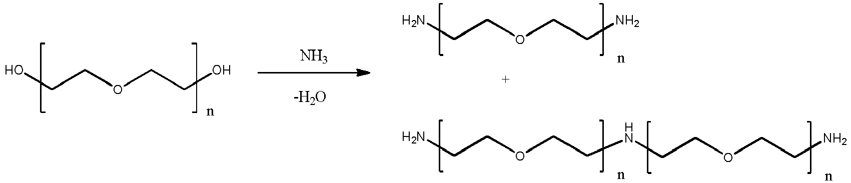

Ein weiteres Problem, das bei der heterogen-katalysierten Aminierung von Polyetherolen, insbesondere bei Polyethylen- und Polypropylenglykolderivaten, beobachtet wird, ist die Zersetzung dieser Ether bei den vorstehend beschriebenen Reaktionsbedingungen, da insbesondere die hohen Temperaturen und ein Wasserstoffstützdruck notwendig sind. Unter diesen Reaktionsbedingungen entstehen gasförmige Zersetzungsprodukte, die spezielle Sicherheitsvorkehrungen notwendig machen.

Die homogen-katalysierte Aminierung von Monoalkoholen mit primären und sekundären Aminen ist seit den 1970er Jahren bekannt, wobei meist Ruthenium- oder Iridiumkatalysatoren beschrieben sind. Die homogen-katalysierte Aminierung läuft, im Vergleich zu heterogen-katalysierten Reaktionen, bei deutlich niedrigeren Temperaturen von 100 bis 150°C ab. Die Umsetzung von Monoalkoholen mit primären und sekundären Aminen ist beispielsweise in folgenden Veröffentlichungen beschrieben: US 3,708,539; Y. Watanabe, Y. Tsuji, Y. Ohsugi, Tetrahedron Lett. 1981, 22, 2667-2670; S. Bähn, S. Imm, K. Mevius, L. Neubert, A. Tillack, J. M. J. Williams, M. Beller, Chem. Eur. J. 2010, DOI: 10.1002/chem.200903144; A. Tillack, D. Hollmann, D. Michalik, M. Beller, Tetrahedron Lett. 2006, 47, 8881-8885; D. Hollmann, S. Bähn, A. Tillack, M. Beller, Angew. Chem. Int. Ed. 2007, 46, 8291-8294; A. Tillack, D. Hollmann, K. Mevius, D. Michalik, S. Bähn, M. Beller, Eur. J. Org. Chem. 2008, 4745-4750; M. H. S. A. Hamid, C. L. Allen, G. W. Lamb, A. C. Maxwell, H. C. Maytum, A. J. A. Watson, J. M. J. Williams, J. Am. Chem. Soc. 2009, 131, 1766-1774; O. Saidi, A. J. Blacker, M. M. Farah, S. P. Marsden, J. M. J. Williams, Chem. Commun. 2010, 46, 1541-1543; A. Tillack, D. Hollmann, D. Michalik, M. Beller, Tet. Lett. 2006, 47, 8881-8885; A. Del Zlotto, W. Baratta, M. Sandri, G. Verardo, P. Rigo, Eur. J. Org. Chem. 2004, 524-529; A. Fujita, Z. Li, N. Ozeki, R. Yamaguchi, Tetrahedron Lett. 2003, 44, 2687-2690; Y. Watanabe, Y. Morisaki, T. Kondo, T. Mitsudo J. Org. Chem. 1996, 61, 4214-4218, B. Blank, M. Madalska, R. Kempe, Adv. Synth. Catal. 2008, 350, 749-750, A. Martinez-Asencio, D. J. Ramon, M. Yus, Tetrahedron Lett. 2010, 51, 325-327. Der größte Nachteil der vorstehend beschriebenen Systeme ist, dass mit diesen Verfahren nur die Aminierung von Monoalkoholen mit primären und sekundären Aminen möglich ist. Die Umsetzung von Alkoholen mit Ammoniak, welches die wirtschaftlich attraktivste Aminierungsreaktion darstellt, wird in diesen Arbeiten nicht beschrieben.

Die Aminierung von Diolen mit sekundären Aminen mittels homogenen Iridium- und Rutheniumkatalysatoren zu Aminoalkoholen und linearen Diaminen mit tertiären Aminogruppen ist beispielsweise in EP 239 934; J. A. Marsella, J. Org. Chem. 1987, 52, 467-468; US 4,855,425; K.-T. Huh, Bull. Kor. Chem. Soc. 1990, 11, 45-49; N. Andrushko, V. Andrushko, P. Roose, K. Moonen, A. Börner, ChemCatChem, 2010, 2, 640-643 und S. Bähn, A. Tillack, S. Imm, K. Mevius, D. Michalik, D. Hollmann, L. Neubert, M. Beller, ChemSusChem 2009, 2, 551-557 beschrieben. Die Aminierung wird in diesen Arbeiten bei 100-180°C durchgeführt.

J. A. Marsella, J. Organomet. Chem. 1991, 407, 97-105 und B. Blank, S. Michlik, R. Kempe, Adv. Synth. Catal. 2009, 351, 2903-2911; G. Jenner, G. Bitsi, J. Mol. Cat, 1988, 45, 165-168; Y. Z. Youn, D. Y. Lee, B. W. Woo, J. G. Shim, S. A. Chae, S. C. Shim, J. Mol. Cat, 1993, 79, 39-45; K. I. Fujita, R. Yamaguchi, Synlett, 2005, 4, 560-571; K.I. Fujii, R. Yamaguchi, Org. Lett. 2004, 20, 3525-3528; K. I. Fujita, K. Yamamoto, R. Yamaguchi, Org. Lett. 2002, 16, 2691-2694; A. Nova, D. Balcells, N. D. Schley, G. E. Dobereiner, R. H. Crabtree, O. Eisenstein, Organometallics DOI: 10.1021/om101015u; und M. H. S. A. Hamid, C. L. Allen, G. W. Lamb, A. C. Maxwell, H. C. Maytum, A. J. A. Watson, J. M. J. Williams, J. Am. Chem. Soc. 2009, 131, 1766-1774 und O. Saidi, A. J. Blacker, G. W. Lamb, S. P. Marsden, J. E. Taylor, J. M. J. Williams, Org. Proc. Res. Dev. 2010, 14, 1046-1049 beschreiben die Aminierung von Diolen sowie Alkanolaminen mit primären Aminen unter Verwendung von homogengelösten Ruthenium- und Iridium-basierten Übergangsmetallkatalysatoren. Hierbei bilden sich jedoch die zyklischen Verbindungen und nicht die gewünschten linearen Diamine. Die wirtschaftlich attraktive Aminierung von Diolen mit Ammoniak zu primären Aminen ist mit diesen Systemen nicht möglich.

S. Imm, S. Bähn, L. Neubert, H. Neumann, M. Beller, Angew. Chem. 2010, 122, 8303-8306 und D. Pingen, C. Müller, D. Vogt, Angew. Chem. 2010, 122, 8307-8310 beschreiben die mit Rutheniumkatalysatoren homogen-katalysierte Aminierung von sekundären Alkoholen wie Cyclohexanol mit Ammoniak. EP 0 320 269 A2 offenbart die Palladium katalysierte Aminierung von primären Allylmonoalkohlen mit Ammoniak unter Bildung primärer Allylamine. WO 2010/018570 und C. Gunanathan, D. Milstein, Angew. Chem. Int. Ed. 2008, 47, 8661-8664 beschreiben die Aminierung von primären Monoalkoholen mit Ammoniak zu primären Monoaminen mit Hilfe von Ruthenium-Phosphan-Komplexen. Die Aminierung von primären Di-, Tri- und Polyolen ist in diesen Arbeiten nicht beschrieben.

R. Kawahara, K.I. Fujita, R. Yamaguchi, J. Am. Chem. Soc. DOI: 10.1021/ja107274w beschreibt die Aminierung primärer Monoalkoholen und Triole mit Ammoniak unter Verwendung eines Iridiumkatalysators, der als Liganden Cp* (1,2,3,4,5-Pentamethylcyclopentadienyl) und Ammoniak aufweist. Mit dem dort beschriebenen Katalysatorsystem werden beim Umsatz primärer Monoalkohole mit Ammoniak jedoch ausschließlich die unerwünschten tertiären Amine erhalten. Die Umsetzung von Glycerin mit Ammoniak führt ausschließlich zum unerwünschten bizyklischen Quinolizidin.

EP 0 234 401 A1 beschreibt die Umsetzung von Diethylenglykol mit Ammoniak in Gegenwart einer Rutheniumcarbonylverbindung. Bei dem in EP 0 234 401 A1 beschriebene Verfahren bilden sich lediglich das Monoaminierungsprodukt (Monoethanolamin), die sekundären und tertiären Amine (Di- und Triethanolamin) und zyklische Produkte (N-(Hydroxyethyl)piperazin und N,N'-Bis(hydroxyethyl)piperazin). Das gewünschte 1,2-Diaminoethan wird bei diesem Verfahren nicht erhalten.

Alle vorstehend beschriebenen Verfahren zur Umsetzung von Di- und Triolen haben den Nachteil, dass sich als Hauptprodukte die unerwünschten sekundären, tertiären und zyklischen Amine bilden. Teilweise bilden sich in untergeordneter Rolle auch Monoaminierungsprodukte wie Alkanolamine. Die gewünschten primären Diamine, Triamine und Polyamine sind auf diese Weise nicht zugänglich.

Aufgabe der vorliegenden Erfindung ist es, ein Verfahren zur Herstellung von primären Di-, Tri- und Polyaminen durch Alkoholaminierung von Di-, Tri- und Polyolen sowie von Alkanolaminen mit Ammoniak unter Wasserabspaltung bereitzustellen.

Gelöst wird die Aufgabe durch ein Verfahren nach Anspruch 1 zur Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak unter Wasserabspaltung, wobei die Reaktion homogen-katalysiert in Gegenwart mindestens eines Komplexkatalysators, der mindestens ein Element ausgewählt aus den Gruppen 8, 9 und 10 des Periodensystems sowie mindestens einen Donorliganden, insbesondere einen Phosphordonorliganden, enthält, durchgeführt wird.

Überraschend wurde festgestellt, dass sich mit den im erfindungsgemäßen Verfahren eingesetzten Komplexkatalysatoren, die mindestens ein Element ausgewählt aus der Gruppe 8, 9 und 10 des Periodensystems sowie mindestens einen Donorliganden, insbesondere einen Phosphordonorliganden, enthalten, primäre Di-, Tri- und Oligoamine durch die homogen-katalysierte Aminierung von Diolen, Triolen und Polyolen sowie Alkanolaminen mit Ammoniak unter Wasserabspaltung erhalten lassen. Das erfindungsgemäße Verfahren hat den Vorteil, dass es primäre Di-, Tri- und Polyamine gegenüber den im Stand der Technik beschriebenen Verfahren in deutlich verbesserten Ausbeuten liefert. Darüber hinaus wird die Bildung unerwünschter Nebenprodukte wie sekundärer und tertiärer Amine sowie zyklischer Amine weitestgehend unterdrückt.

### Edukte

Im erfindungsgemäßen Verfahren werden Edukte eingesetzt, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen.

In einer weiteren Ausführungsform werden im erfindungsgemäßen Verfahren Edukte eingesetzt, in denen (-X) ausgewählt ist aus funktionellen Gruppen der Formeln (-CH₂-OH) und (-CH₂-NH₂). Die Edukte weisen dann mindestens eine funktionelle Einheit der Formel (-CH₂-OH) und mindestens eine weitere funktionelle Einheit ausgewählt aus funktionellen Einheiten der Formel (-CH₂-OH) und (-CH₂-NH₂) auf.

Als Edukte sind praktisch alle Alkohole geeignet, die die vorstehend genannten Voraussetzungen erfüllen. Die Alkohole können geradkettig, verzweigt oder zyklisch sein. Die Alkohole können darüber hinaus Substituenten tragen, die sich unter den Reaktionsbedingungen der Alkoholaminierung inert verhalten, beispielsweise Alkoxy, Alkenyloxy, Alkylamino, Dialkylamino und Halogene (F, Cl, Br, I).

Geeignete Edukte, die im erfindungsgemäßen Verfahren eingesetzt werden können, sind beispielsweise Diole, Triole, Polyole und Alkanolamine, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen.

Darüber hinaus sind Diole, Triole, Polyole und Alkanolamine geeignet, die mindestens eine funktionelle Einheit der Formel (-CH₂-OH) und mindestens eine weitere funktionelle Einheit ausgewählt aus funktionellen Einheiten der Formel (-CH₂-OH) und (-CH₂-NH₂) aufweisen.

Als Edukte können alle bekannten Diole eingesetzt werden, die mindestens eine funktionelle Gruppe der Formel (-CH₂-OH) aufweisen. Beispiele für Diole, die als Edukte in das erfindungsgemäße Verfahren eingesetzt werden können, sind 1,2-Ethandiol (Ethylenglykol), 1,2-Propandiol (1,2-Propylenglykol), 1,3-Propandiol (1,3-Propylenglykol), 1,4-Butandiol (1,4-Butylenglykol), 1,2-Butandiol (1,2-Butylenglykol), 2,3-Butandiol, 2-Methyl-1,3-propandiol, 2,2-Dimethyl-1,3-propandiol (Neopentylglykol), 1,5-Pentandiol, 1,2-Pentandiol, 1,6-Hexandiol, 1,2-Hexandiol, 1,7-Heptandiol, 1,2-Heptandiol, 1,8-Oktandiol, 1,2-Oktandiol, 1,9-Nonandiol, 1,2-Nonandiol, 2,4-Dimethyl-2,5-hexandiol, Hydroxypivalinsäureneopentylglykolester, Diethylenglykol, Triethylenglykol, 2-Buten-1,4-diol, 2-Butin-1,4-diol, Polyethylenglykole, Polypropylenglykole wie 1,2-Polypropylenglykol und 1,3 Polypropylenglykol, Polytetrahydrofuran (Polytetramethylenglykol), Diethanolamin, 1,4-Bis-(2-hydroxyethyl)piperazin, Diisopropanolamin, N-Butyldiethanolamin, N-Methyldiethanolamin, 1,10-Decandiol, 1,12-Dodecandiol, 2,5-(Dimethanol)furan, 1,4-Bis(Hydroxymethyl)cyclohexan und C₃₆-Diol (Gemisch aus Isomeren von Alkoholen der Summenformel C₃₆H₇₄O₂). 2,5-(Dimethanol)furan wird auch als 2,5-Bis(hydroxymethyl)furan bezeichnet.

Bevorzugt sind Diole, die zwei funktionelle Gruppen der Formel (-CH₂-OH) aufweisen.

Insbesondere bevorzugte Diole sind 2-Ethandiol (Ethylenglykol), 1,2-Propandiol (1,2-Propylenglykol), 1,3-Propandiol (1,3-Propylenglykol), 1,4-Butandiol (1,4-Butylenglykol), 2-Methyl-1,3-propandiol, 2,2-Dimethyl-1,3-propandiol (Neopentylglykol), 1,5-Pentandiol, 1,6-Hexandiol, 1,7-Heptandiol, 1,8-Oktandiol, 1,9-Nonandiol, Diethylenglykol, Triethylenglykol, Polyethylenglykole, Polypropylenglykole, Polytetrahydrofuran, Diethanolamin, 1,10-Decandiol, 1,12-Dodecandiol, 2,5-(Dimethanol)furan und C₃₆-Diol (Gemisch aus Isomeren von Alkoholen der Summenformel C₃₆H₇₄O₂).

Als Diole am meisten bevorzugt sind Ethylenglykol, Diethanolamin, Polytetrahydrofuran, Diethylenglykol, 2,5-(Dimethanol)furan und 1,4-Butandiol.

Als Edukte können alle bekannten Triole eingesetzt werden, die mindestens eine funktionelle Gruppe der Formel (-CH₂-OH) aufweisen. Beispiele für Triole, die als Edukte in das erfindungsgemäße Verfahren eingesetzt werden können, sind Glycerin, Trimethylolpropan und Triethanolamin.

Bevorzugt sind Triole, die mindestens zwei funktionelle Gruppen der Formel (-CH₂-OH) aufweisen.

Insbesondere bevorzugte Triole sind Glycerin, Trimethylolpropan und Triethanolamin.

Als Edukte können alle bekannten Polyole eingesetzt werden, die mindestens eine funktionelle Gruppe der Formel (-CH₂-OH) aufweisen. Beispiele für Polyole, die als Edukte in das erfindungsgemäße Verfahren eingesetzt werden können, sind 2,2-Bis(hydroxymethyl)-1,3-propandiol (Pentaerythrit), Zucker und Polymere wie beispielsweise Glucose, Mannose, Fructose, Ribose, Desoxyribose, Galactose, Fucose, Rhamnose, Saccharose, Lactose, Cellolbiose, Maltose und Amylose, Cellulose, Xanthan und Polyvinylalkohole.

Bevorzugt sind Polyole, die mindestens zwei funktionelle Gruppen der Formel (-CH₂-OH) aufweisen.

Als Edukte können alle bekannten Alkanolamine eingesetzt werden, die mindestens eine primäre Hydroxylgruppen (-CH₂-OH) und mindestens eine primäre Aminogruppe (-NH₂) aufweisen. Beispiele für Alkanolamine, die als Edukte in das erfindungsgemäße Verfahren eingesetzt werden können, sind Monoaminoethanol, 3-Aminopropan-1-ol, 2-Aminopropan-1-ol, 4-Aminobutan-1-ol, 2-Aminobutan-1-ol, 3-Aminobutan-1-ol, 5-Aminopentan-1-ol, 2-Aminopentan-1-ol, 6-Aminohexan-1-ol, 2-Aminohexan-1ol, 7-Aminoheptan-1-ol, 2-Aminoheptan-1-ol, 8-Aminooctan-1-ol, 2-Aminooctan-1-ol, N-(2-Aminoethyl)ethanolamin, Monoaminodiethylenglykol (2-(2-Aminoethoxy)ethanol), N-(2-Hydroxyethyl)-1,3-propandiamin und 3- (2-Hydroxyethyl)amino-1-propanol.

Bevorzugt sind Alkanolamine, die mindestens eine primäre Hydroxylgruppen (-CH₂-OH) und mindestens eine primäre Aminogruppe der Formel (-CH₂-NH₂) aufweisen.

Insbesondere bevorzugte Alkanolamine sind Monoaminoethanol, Monoaminodiethylenglykol (2-(2-Aminoethoxy)ethanol), 2-Aminopropan-1-ol, 3-Aminopropan-1-ol und 4-Aminobutan-1-ol.

### Komplexkatalysator

Im erfindungsgemäßen Verfahren wird mindestens ein Komplexkatalysator, der mindestens ein Element ausgewählt aus den Gruppen 8, 9 und 10 des Periodensystems (Nomenklatur gemäß IUPAC) sowie mindestens einen Donorliganden enthält, eingesetzt. Die Elemente der Gruppe 8, 9 und 10 des Periodensystems umfassen Eisen, Cobalt, Nickel, Ruthenium, Rhodium, Palladium, Osmium, Iridium und Platin. Bevorzugt sind Komplexkatalysatoren, die mindestens ein Element ausgewählt aus Ruthenium und Iridium enthalten.

In einer Ausführungsform wird das erfindungsgemäße Verfahren homogen-katalysiert in Gegenwart mindestens eines Komplexkatalysators der allgemeinen Formel (I) durchgeführt: 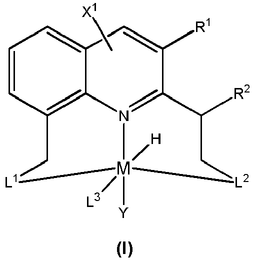 in der
- L¹ und L²: unabhängig voneinander Phosphin (PR^{a}R^{b}), Amin (NR^{a}R^{b}), Sulfid, SH, Sulfoxid (S(=O)R), C₅-C₁₀-Heteroaryl enthaltend mindestens ein Heteroatom ausgewählt aus Stickstoff (N), Sauerstoff (O) und Schwefel (S), Arsin (AsR^{a}R^{b}), Stiban (SbR^{a}R^{b}) oder N-heterozyklische Carbene der Formel (II) oder (III): 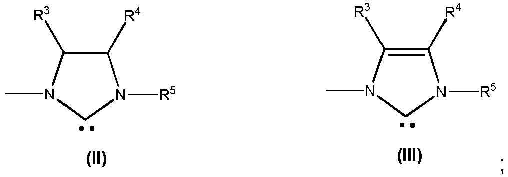
- L³: einzähniger Zweielektronendonor ausgewählt aus der Gruppe Kohlenmonoxid (CO), PR^{a}R^{b}R^{c}, NO⁺, AsR^{a}R^{b}R^{c}, SbR^{a}R^{b}R^{c}, SR^{a}R^{b}, Nitril (RCN), Isonitril (RNC), Stickstoff (N₂), Phosphortrifluorid (PF₃), Kohlenstoffmonosulfid (CS), Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S, wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit, wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem oder zumindest monosubstituiertem C₁-C₁₀-Alkoxy, C₁-C₁₀-Alkyl, C₃-C₁₀)-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten.

Hierbei ist darauf hinzuweisen, dass der Komplexkatalysator der Formel (I) für den Fall, dass Y ein neutrales Molekül aus der Gruppe NH₃, NR₃, R₂NSO₂R ist und M ausgewählt ist aus der Gruppe Ruthenium, Nickel, Palladium, Platin und Eisen, eine positive Ladung trägt.

In einer bevorzugten Ausführungsform wird das erfindungsgemäße Verfahren in Gegenwart mindestens eines homogen gelösten Komplexkatalysators der Formel (I) durchgeführt, wobei die Substituenten folgende Bedeutung haben:
- L¹ und L²,: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S;
- L³: einzähniger Zweielektronendonor, ausgewählt aus der Gruppe CO, PR^{a}R^{b}R^{c}, NO⁺, RCN, RNC, N₂, PF₃, CS, Pyridin, Thiophen und Tetrahydrothiophen;
- R¹ und R²: beide Wasserstoff oder gemeinsam mit den Kohlenstoffatomen, an die sie gebunden sind ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S;
- Y: monoanionischer Ligand ausgewählt aus der Gruppe H, F, Cl, Br, OCOR, OCOCF₃, OSO₂R, OSO₂CF₃, CN, OH, OR oder N(R)₂;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium.

In einer weiteren bevorzugten Ausführungsform wird das erfindungsgemäße Verfahren in Gegenwart mindestens eines homogen gelösten Komplexkatalysators durchgeführt, wobei R¹ und R² beide Wasserstoff sind und der Komplexkatalysator ein Katalysator der Formel (IV) ist: 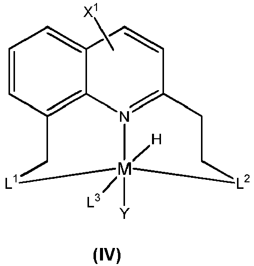 und X¹, L¹, L², L³ und Y die vorstehend genannten Bedeutungen aufweisen.

In einer weiteren bevorzugten Ausführungsform wird das erfindungsgemäßen Verfahrens in Gegenwart mindestens eines homogen gelösten Komplexkatalysator durchgeführt, wobei R¹ und R² gemeinsam mit den Kohlenstoffatomen, an die sie gebunden sind einen Phenylring bilden, der zusammen mit der Chinolinyleinheit der Formel (I) eine Acridinyleinheit bildet und der Komplexkatalysator ein Katalysator der Formel (V) ist: 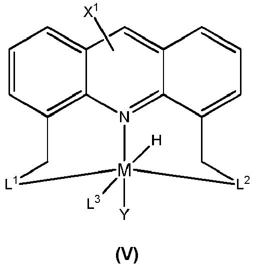 und X¹, L¹, L², L³ und Y die vorstehend genannten Bedeutungen aufweisen.

Nachfolgend werden exemplarisch einige Komplexkatalysatoren (Formeln (VI), (VII), (VIII), (IX), (X), (XI), (XII) und (XIII)) aufgeführt, die im erfindungsgemäßen Verfahren eingesetzt werden können:

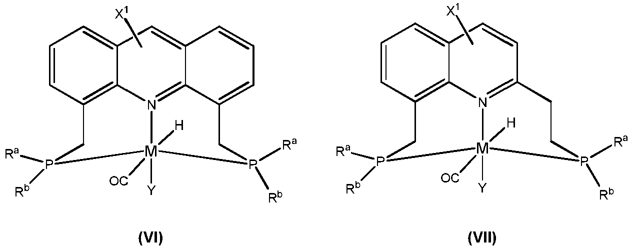

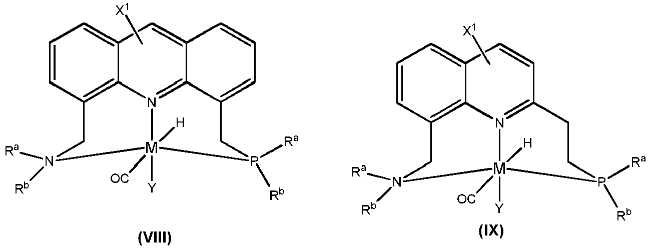

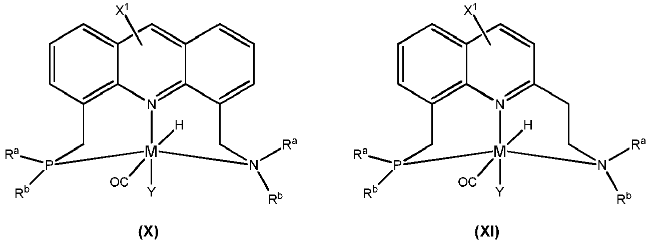

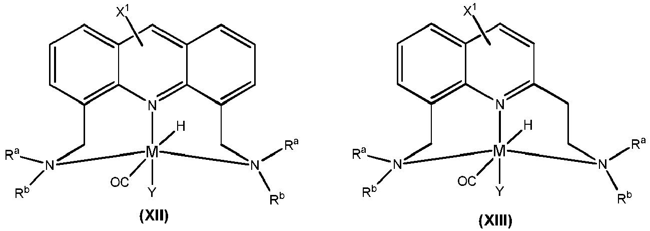

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, OCOR, OCOCF₃, OSO₂R, OSO₂CF₃, CN, OH, OR, N(R)₂;
- R: unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl, C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- X¹: ein, zwei oder drei, Substituenten an einem oder mehreren Atomen der Acridinyleinheit oder ein oder zwei Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, - C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
und
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert.-Butyl, Cyclohexyl, Cyclopentyl, Phenyl oder Mesityl;
- Y: monoanionischer Ligand ausgewählt aus der Gruppe H, F, Cl, Br, OCOCH₃, OCOCF₃, OSO₂CF₃, CN und OH;
- X¹: ein Substituent an einem Atom der Acridinyleinheit oder ein Substituenten an einem Atom der Chinolinyleinheit,
wobei X¹ ausgewählt ist aus der Gruppe bestehend aus Wasserstoff, F, Cl, Br, OH, NH₂, NO₂ -NC(O)R, C(O)NR₂, -OC(O)R, -C(O)OR, CN und Boranderivaten, die aus dem Katalysator der Formel (I) durch Reaktion mit NaBH₄ erhältlich sind und unsubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S;
- M: Ruthenium oder Iridium
bedeuten.

In einer weiteren bevorzugten Ausführungsform wird das erfindungsgemäße Verfahrens in Gegenwart mindestens eines Komplexkatalysator aus der Gruppe der Katalysatoren der Formel (VI), (VII), (VIII), (IX), (X), (XI), (XII) und (XIII) durchgeführt, wobei
- R^{a} und R^{b}: unabhängig voneinander Methyl, Ethyl, Isopropyl, tert. Butyl, Cyclohexyl, Cyclopentyl, Phenyl oder Mesityl;
- Y: monoanionischer Ligand aus der Gruppe H, F, Cl, Br, I, OCOCH₃, OCOCF₃, OSO₂CF₃, CN und OH;
- X¹: Wasserstoff;
und
- M: Ruthenium oder Iridium
bedeuten.

In einer besonders bevorzugten Ausführungsform ist L³ Kohlenmonoxid (CO).

In einer besonders bevorzugten Ausführungsform wird das erfindungsgemäße Verfahren in Gegenwart eines Komplexkatalysators der Formel (XIVa) durchgeführt:

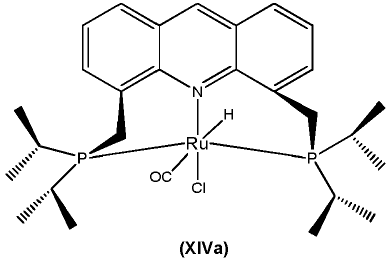

In einer weiteren besonders bevorzugten Ausführungsform wird das erfindungsgemäße Verfahren in Gegenwart eines Komplexkatalysators der Formel (XIVb) durchgeführt.

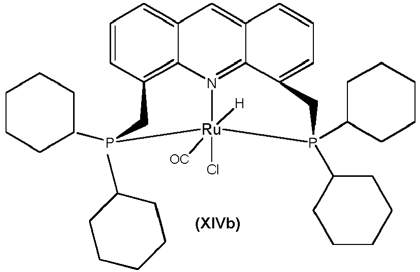

In einer insbesondere bevorzugten Ausführungsform wird das erfindungsgemäße Verfahren in Gegenwart eines Komplexkatalysators der allgemeinen Formel (XIVb) durchgeführt.

In einer weiteren besonders bevorzugten Ausführungsform wird das erfindungsgemäße Verfahrens in Gegenwart mindestens eines homogen gelösten Komplexkatalysator der Formel (XV) durchgeführt, in der R¹, R², R³, L¹, L² und L³ die vorstehend beschriebenen Bedeutungen haben.

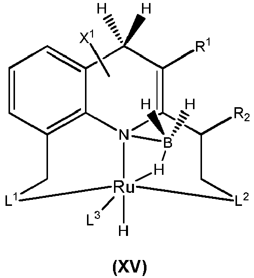

Komplexkatalysatoren der Formel (XV) sind erhältlich durch Umsetzung von Katalysatoren der Formel (I) mit Natriumborhydrid (NaBH₄). Die Umsetzung folgt dabei der allgemeinen Reaktionsgleichung:

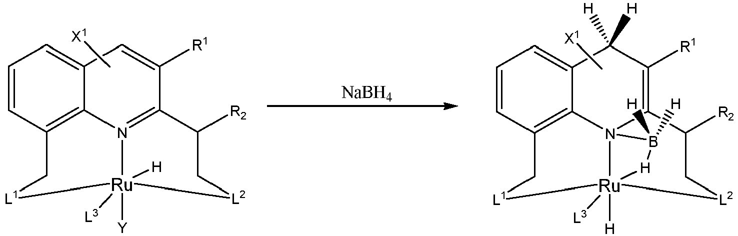

In einer weiteren besonders bevorzugten Ausführungsform wird das erfindungsgemäße Verfahrens in Gegenwart eines Komplexkatalysators der Formel (XVIa) durchgeführt:

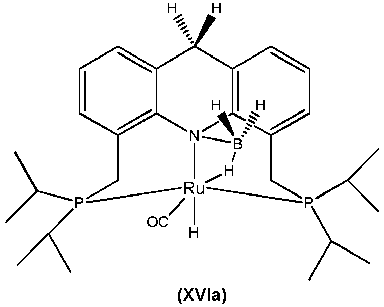

In einer weiteren besonders bevorzugten Ausführungsform wird das erfindungsgemäße Verfahren in Gegenwart eines Komplexkatalysators der Formel (XVIb) durchgeführt.

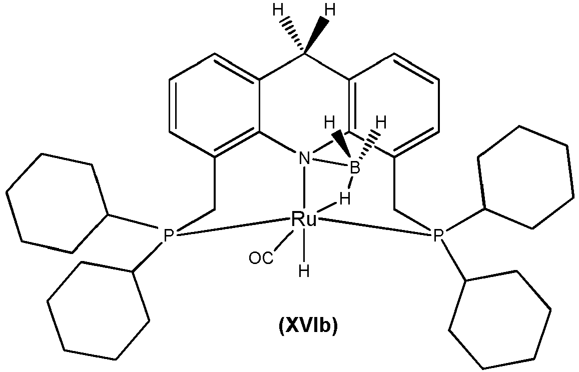

Das Boranderivat der Formel (XVIa) ist gemäß folgender Reaktionsgleichung zugänglich:

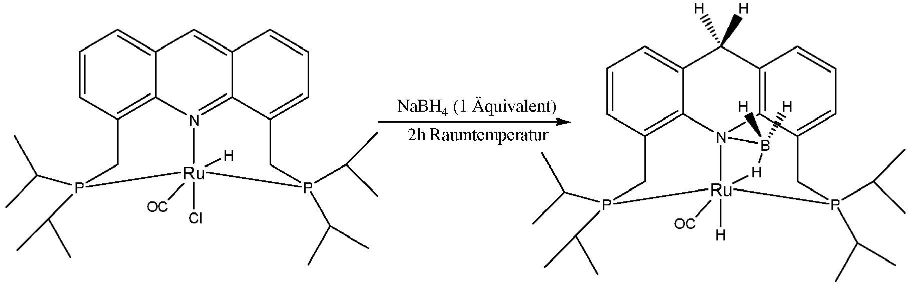

Das Boranderivat der Formel (XVIb) ist gemäß folgender Reaktionsgleichung zugänglich:

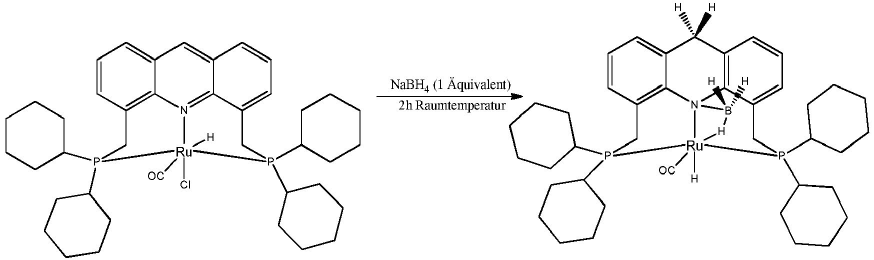

Im Rahmen der vorliegenden Erfindung werden unter C₁-C₁₀-Alkyl verzweigte, unverzweigte, gesättigte und ungesättigte Gruppen verstanden. Bevorzugt sind Alkylgruppen mit 1 bis 6 Kohlenstoffatomen (C₁-C₆-Alkyl). Mehr bevorzugt sind Alkylgruppen mit 1 bis 4 Kohlenstoffatomen (C₁-C₄-Alkyl).

Beispiele für gesättigte Alkylgruppen sind Methyl, Ethyl, n-Propyl, Isopropyl, n-Butyl, Isobutyl, sec.-Butyl, tert.-Butyl, Amyl und Hexyl.

Beispiele für ungesättigte Alkylgruppen (Alkenyl, Alkinyl) sind Vinyl, Allyl, Butenyl, Ethinyl und Propinyl.

Die C₁-C₁₀-Alkylgruppe kann unsubstituiert oder, mit einem oder mehreren Substituenten ausgewählt aus der Gruppe F, Cl, Br, Hydroxy (OH), C₁-C₁₀-Alkoxy, C₅-C₁₀-Aryloxy, C₅-C₁₀-Alkylaryloxy, C₅-C₁₀-Heteroaryloxy enthaltend mindestens ein Heteroatom ausgewählt aus N, O, S, Oxo, C₃-C₁₀-Cycloalkyl, Phenyl, C₅-C₁₀-Heteroaryl enthaltend mindestens ein Heteroatom ausgewählt aus N, O, S, C₅-C₁₀-Heterocyclyl enthaltend mindestens ein Heteroatom ausgewählt aus N, O, S, Naphthyl, Amino, C₁-C₁₀-Alkylamino, C₅-C₁₀-Arylamino, C₅-C₁₀-Heteroarylamino enthaltend mindestens ein Heteroatom ausgewählt aus N, O, S, C₁-C_{10*}Dialkylamino, C₁₀-C₁₂-Diarylamino, C₁₀-C₂₀Alkylarylamino, C₁-C₁₀-Acyl, C₁-C₁₀-Acyloxy, NO₂, C₁-C₁₀-Carboxy, Carbamoyl, Carboxamid, Cyano, Sulfonyl, Sulfonylamino, Sulfinyl, Sulfinylamino, Thiol, C₁-C₁₀-Alkylthiol, C₅-C₁₀-Arylthiol oder C₁-C₁₀-Alkylsulfonyl substituiert sein.

Unter C₃-C₁₀-Cycloalkyl werden vorliegend gesättigte, ungesättigte monozyklische und polyzyklische Gruppen verstanden. Beispiele für C₃-C₁₀-Cycloalkyl sind Cyclopropyl, Cyclobutyl, Cyclopentyl, Cyclohexyl oder Cycloheptyl. Die Cycloalkylgruppen können unsubstituiert oder substituiert sein mit einem oder mehreren Substituenten, wie sie vorstehend zu der Gruppe C₁-C₁₀-Alkyl definiert wurde.

Im Rahmen der vorliegenden Erfindung wird unter C₅-C₁₀-Aryl ein aromatisches Ringsystem mit 5 bis 10 Kohlenstoffen verstanden. Das aromatisches Ringsystem kann monozyklisch oder bizyklisch sein. Beispiele für Arylgruppen sind Phenyl, Naphthyl wie 1-Naphthyl und 2-Naphthyl. Die Arylgruppe kann unsubstituiert oder substituiert sein, mit einem oder mehreren Substituenten wie vorstehend unter C₁-C₁₀-Alkyl definiert.

Im Rahmen der vorliegenden Erfindung wird unter C₅-C₁₀-Heteroaryl ein heteroaromatisches System verstanden, das mindestens ein Heteroatom, ausgewählt aus der Gruppe N, O und S, enthält. Die Heteroarylgruppen können monozyklisch oder bizyklisch sein. Für den Fall, dass Stickstoff ein Ringatom ist, umfasst die vorliegende Erfindung auch N-Oxide der stickstoffenthaltenden Heteroaryle. Beispiele für Heteroaryle sind Thienyl, Benzothienyl, 1-Naphthothienyl, Thianthrenyl, Furyl, Benzofuryl, Pyrrolyl, Imidazolyl, Pyrazolyl, Pyridyl, Pyrazinyl, Pyrimidinyl, Pyridazinyl, Indolyl, Isoindolyl, Indazolyl, Purinyl, Isoquinolinyl, Quinolinyl, Acridinyl, Naphthyridinyl, Quinoxalinyl, Quinazolinyl, Cinnolinyl, Piperidinyl, Carbolinyl, Thiazolyl, Oxazolyl, Isothiazolyl, Isoxazolyl. Die Heteroarylgruppen können unsubstituiert oder substituiert sein, mit einem oder mehreren Substituenten, die vorstehend unter C₁-C₁₀-Alkyl definiert wurden.

Im Rahmen der vorliegenden Erfindung werden unter C₃-C₁₀-Heterocyclyl fünf- bis zehngliedrige Ringsysteme verstanden, die mindestens ein Heteroatom aus der Gruppe N, O und S enthalten. Die Ringsysteme können mono- oder bizyklisch sein. Beispiele für geeignete heterozyklische Ringsysteme sind Piperidinyl, Pyrrolidinyl, Pyrrolinyl, Pyrazolinyl, Pyrazolidinyl, Morpholinyl, Thiomorpholinyl, Pyranyl, Thiopyranyl, Piperazinyl, Indolinyl, Dihydrofuranyl, Tetrahydrofuranyl, Dihydrothiophenyl, Tetrahydrothiophenyl, Dihydropyranyl und Tetrahydropyranyl.

### Alkoholaminierung

Die homogenen Katalysatoren können sowohl direkt in ihrer aktiven Form als auch ausgehend von üblichen Vorstufen unter Zugabe der entsprechenden Liganden erst unter den Reaktionsbedingungen erzeugt werden. Übliche Vorstufen sind beispielsweise [Ru(p-cymene)Cl₂]₂, [Ru(benzol)Cl₂]ₙ, [Ru(CO)₂Cl₂]ₙ, [Ru(CO)₃Cl₂]₂ [Ru(COD)(allyl)], [RuCl₃*H₂O], [Ru(acetylacetonat)₃], [Ru(DMSO)₄Cl₂], [Ru(PPh₃)₃(CO)(H)Cl], [Ru(PPh₃)₃(CO)Cl₂], [Ru(PPh₃)₃(CO)(H)₂], [Ru(PPh₃)₃Cl₂], [Ru(cyclopentadienyl)(PPh₃)₂Cl], [Ru(cyclopentadienyl)(CO)₂Cl], [Ru(cyclopentadienyl)(CO)₂H], [Ru(cyclopentadienyl)(CO)₂]₂, [Ru(pentamethylcyclopentadienyl)(CO)₂Cl], [Ru(pentamethylcylcopentadienyl)(CO)₂H], [Ru(pentamethylcyclopentadienyl)(CO)₂]₂, [Ru(indenyl)(CO)₂Cl], [Ru(indenyl)(CO)₂H], [Ru(indenyl)(CO)₂]₂, Ruthenocen, [Ru(binap)Cl₂], [Ru(bipyridin)₂Cl₂*2H₂O], [Ru(COD)Cl₂]₂, [Ru(pentamethylcyclopentadienyl)(COD)CI], [Ru₃(CO)₁₂], [Ru(tetraphenylhydroxy-cyclopentadienyl)(CO)₂H], [Ru(PMe₃)₄(H)₂], [Ru(PEt₃)₄(H)₂], [Ru(P*n*Pr₃)₄(H)₂], [Ru(P*n*Bu₃)₄(H)₂], [Ru(P*n*Octyl₃)₄(H)₂], [IrCl₃*H₂O], KIrCl₄, K₃IrCl₆, [Ir(COD)Cl]₂, [Ir(cycloocten)₂Cl]₂, [Ir(ethen)₂Cl]₂, [Ir(cyclopentadienyl)Cl₂]₂, [Ir(pentamethylcyclopentadienyl)Cl₂]₂, [Ir(cylopentadienyl)(CO)_{2]}, [Ir(pentamethylcyclopentadienyl)(CO)₂], [Ir(PPh₃)₂(CO)(H)], [Ir(PPh₃)₂(CO)(Cl)], [Ir(PPh₃)₃(CI)].

Unter homogen-katalysiert wird im Rahmen der vorliegenden Erfindung verstanden, dass der katalytisch-aktive Teil des Komplexkatalysators zumindest teilweise im flüssigen Reaktionsmedium gelöst vorliegt. In einer bevorzugten Ausführungsform liegen mindestens 90 % des im Verfahren eingesetzten Komplexkatalysators im flüssigen Reaktionsmedium gelöst vor, mehr bevorzugt mindestens 95 %, insbesondere bevorzugt mehr als 99 %, am meisten bevorzugt liegt der Komplexkatalysator vollständig gelöst in flüssigen Reaktionsmedium vor (100%), jeweils bezogen auf die Gesamtmenge im flüssigen Reaktionsmedium.

Die Menge der Metallkomponente des Katalysators, bevorzugt Ruthenium oder Iridium, beträgt im Allgemeinen 0,1 bis 5000 Gewichts-ppm, jeweils bezogen auf das gesamte flüssige Reaktionsmedium.

Die Reaktion erfolgt in der Flüssigphase im Allgemeinen bei einer Temperatur von 20 bis 250°C. Bevorzugt wird das erfindungsgemäße Verfahren bei Temperaturen im Bereich von 100 °C bis 200 °C, besonders bevorzugt im Bereich von 110 bis 160°C durchgeführt.

Die Reaktion kann im Allgemeinen bei einem Gesamtdruck von 0,1 bis 20 MPa absolut, der sowohl der Eigendruck des Lösungsmittels bei der Reaktionstemperatur als auch der Druck eines Gases wie Stickstoff, Argon oder Wasserstoff sein kann, durchgeführt werden. Bevorzugt wird das erfindungsgemäße Verfahren bei einem Gesamtdruck im Bereich von 0,5 bis 10 MPa absolut, insbesondere bevorzugt bei einem Gesamtdruck im Bereich von 1 bis 6 MPa absolut durchgeführt.

Die mittlere Reaktionszeit beträgt im Allgemeinen 15 Minuten bis 100 Stunden.

Das Aminierungsmittel (Ammoniak) kann bezüglich der zu aminierenden Hydroxylgruppen in stöchiometrischen, unterstöchiometrischen oder überstöchiometrischen Mengen eingesetzt werden.

In einer bevorzugten Ausführungsform wird Ammoniak mit einem 1- bis 250-fachen, bevorzugt mit einem 2- bis 100-fachen, insbesondere mit einem 1,5- bis 10-fachen molaren Überschuss pro Mol im Edukt umzusetzender Hydroxylgruppen eingesetzt. Es sind auch höhere Überschüsse an Ammoniak möglich.

Das erfindungsgemäße Verfahren kann sowohl in einem Lösungsmittel als auch ohne Lösungsmittel durchgeführt werden. Als Lösungsmittel eignen sich polare und unpolare Lösungsmittel, die in Reinform oder in Mischungen eingesetzt werden können. Beispielsweise kann im erfindungsgemäßen Verfahren nur ein unpolares oder ein polares Lösungsmittel eingesetzt werden. Es ist auch möglich, Mischungen von zwei oder mehr polaren Lösungsmitteln oder Mischungen von zwei oder mehr unpolaren Lösungsmitteln oder Mischungen aus einem oder mehr polaren mit einem oder mehr unpolaren Lösungsmitteln einzusetzen. Auch das Produkt kann als Lösungsmittel in Reinform oder in Mischungen mit polaren oder mit unpolaren Lösungsmittel eingesetzt werden.

Als unpolare Lösungsmittel eignen sich beispielsweise gesättigte und ungesättigte Kohlenwasserstoffe wie Hexan, Heptan, Oktan, Cyclohexan, Benzol, Toluol, Xylol und Mesitylen, und lineare und zyklische Ether wie THF, Diethylether, 1,4-Dioxan, MTBE (tert-Butylmethylether), Diglyme und 1,2-Dimethoxyethan. Bevorzugt werden Toluol, Xylole oder Mesitylen eingesetzt.

Als polare Lösungsmittel eignen sich beispielsweise Wasser, Dimethylformamid, Formamid, tert-Amylalkohol, tert-Butanol und Acetonitril. Bevorzugt wird Wasser eingesetzt. Das Wasser kann sowohl vor der Reaktion zugesetzt werden, bei der Reaktion als Reaktionswasser entstehen oder auch nach der Reaktion zusätzlich zum Reaktionswasser zugesetzt werden. Ein weiteres bevorzugtes Lösungsmittel ist tert-Amylalkohol. Bevorzugt wird ein Gemisch aus tert-Amylalkohol und Wasser eingesetzt.

Für die Umsetzung in der Flüssigphase leitet man Ammoniak, das mindestens eine funktionelle Gruppe der Formel (-CH₂-OH) und mindestens eine weitere funktionelle Gruppe der Formel (-X) aufweisende Edukt, gegebenenfalls zusammen mit einem oder mehreren Lösungsmitteln, zusammen mit dem Komplexkatalysator in einen Reaktor. Die Zuleitung von Ammoniak, Edukt, gegebenenfalls Lösungsmittel und Komplexkatalysator kann dabei simultan oder getrennt voneinander erfolgen. Die Reaktion kann dabei kontinuierlich, in semibatch-Fahrweise, in batch-Fahrweise, rückvermischt in Produkt als Lösungsmittel oder im geraden Durchgang nicht rückvermischt durchgeführt werden.

Für das erfindungsgemäße Verfahren können prinzipiell alle Reaktoren eingesetzt werden, welche für gas/flüssig-Reaktionen unter der gegebenen Temperatur und dem gegebenen Druck grundsätzlich geeignet sind. Geeignete Standardreaktoren für gas/flüssig- und für flüssig/flüssig-Reaktionssyteme sind beispielsweise in K.D. Henkel, "Reactor Types and Their Industrial Applications", in Ullmann's Encyclopedia of Industrial Chemistry, 2005, Wiley-VCH Verlag GmbH & Co. KGaA, DOI: 10.1002/14356007.b04_087, Kapitel 3.3 "Reactors for gas-liquid reactions" angegeben. Als Beispiele seien genannt Rührkesselreaktoren, Rohrreaktoren oder Blasensäulenreaktoren.

Bei der Aminierungsreaktion wird mindestens eine primäre Hydroxylgruppe (-CH₂-OH) des Edukts mit Ammoniak zu einer primären Aminogruppe (-CH₂-NH₂) umgesetzt, wobei sich jeweils ein Mol Reaktionswasser pro Mol umgesetzter Hydroxylgruppe bildet.

So bilden sich bei der Umsetzung von Alkanolaminen, die nur eine primäre Hydroxylgruppe (-CH₂-OH) aufweisen, die entsprechenden Diamine. Die Umsetzung von Monoaminoethanol führt somit zum entsprechenden 1,2-Diaminoethan.

Bei der Reaktion von Edukten, die neben der funktionellen Gruppe der Formel (-CH₂-OH) eine weitere Hydroxylgruppe aufweisen (Diole), werden beide Hydroxylgruppen mit Ammoniak zu den entsprechenden primären Diaminen umgesetzt. Die Umsetzung von 1,2-Ethylenglykol führt somit zum entsprechenden 1,2-Diaminoethan. Die Umsetzung von 2,5-(Dimethanol)furan führt somit zu 2,5-(Bisaminomethyl)furan.

Bei der Reaktion von Edukten, die neben der funktionellen Gruppe der Formel (-CH₂-OH) zwei weitere Hydroxylgruppe aufweisen (Triole), werden zwei oder drei Hydroxylgruppen mit Ammoniak zu den entsprechenden primären Diaminen oder Triaminen umgesetzt. Die Bildung von Diaminen oder Triaminen kann dabei durch die Menge des eingesetzten Ammoniaks und durch die Reaktionsbedingungen gesteuert werden. Die Umsetzung von Glycerin führt somit zum entsprechenden 1,2-Diaminopropanol oder zum 1,2,3-Triaminopropan.

Bei der Reaktion von Edukten, die neben der funktionellen Gruppe der Formel (-CH₂-OH) mehr als drei weitere Hydroxylgruppe aufweisen (Polyole), werden zwei, drei oder mehr Hydroxylgruppen mit Ammoniak zu den entsprechenden primären Diaminen, Triaminen oder Polyaminen umgesetzt. Die Bildung der entsprechenden primären Diaminen, Triaminen oder Polyaminen kann dabei durch die Menge des eingesetzten Ammoniaks und durch die Reaktionsbedingungen gesteuert werden.

Der bei der Umsetzung entstehende Reaktionsaustrag enthält im Allgemeinen die entsprechenden Aminierungsprodukte, gegebenenfalls das eine oder die mehreren Lösungsmittel, den Komplexkatalysator, gegebenenfalls nicht umgesetzte Edukte und Ammoniak sowie das entstandene Reaktionswasser.

Aus dem Reaktionsaustrag werden der gegebenenfalls vorhandene überschüssige Ammoniak, das gegebenenfalls vorhandene Lösungsmittel, der Komplexkatalysator und das Reaktionswasser entfernt. Das erhaltene Aminierungsprodukt kann weiter aufgearbeitet werden. Der überschüssige Ammoniak, der Komplexkatalysator, gegebenenfalls das oder die Lösungsmittel und gegebenenfalls nicht umgesetzte Edukte können in die Aminierungsreaktion zurückgeführt werden.

Wird die Aminierungsreaktion ohne Lösungsmittel durchgeführt, so ist der homogene Komplexkatalysator nach der Reaktion im Produkt gelöst. Dieser kann im Produkt verbleiben oder durch eine geeignete Methode aus diesem abgetrennt werden. Möglichkeiten zur Abtrennung des Katalysators sind zum Beispiel das Auswaschen mit einem nicht mit dem Produkt mischbaren Lösungsmittel, in welchem sich der Katalysator durch geeignete Wahl der Liganden besser löst als im Produkt. Gegebenenfalls wird der Katalysator durch mehrstufige Extraktion aus dem Produkt abgereichert. Als Extraktionsmittel wird bevorzugt ein auch für die Zielreaktion geeignetes Lösungsmittel wie Toluol, Benzol, Xylole, Alkane, wie Hexane, Heptane und Oktane, und azyklische oder zyklische Ether, wie Diethylether und Tetrahydrofuran eingesetzt, welches nach dem Einengen zusammen mit dem extrahierten Katalysator wieder für die Umsetzung eingesetzt werden kann. Möglich ist auch die Entfernung des Katalysators mit einem geeigneten Absorbermaterial. Eine Abtrennung kann auch durch Zufügen von Wasser zu der Produktphase erfolgen, falls die Reaktion in einem mit Wasser nicht mischbaren Lösungsmittel durchgeführt wird. Löst sich der Katalysator dabei bevorzugt in dem Lösungsmittel, kann er mit dem Lösungsmittel von der wässrigen Produktphase abgetrennt und gegebenenfalls erneut eingesetzt werden. Dies kann durch Wahl geeigneter Liganden bewirkt werden. Die resultierenden wässrigen Di-, Tri-, oder Polyamine können direkt als technische Aminlösungen eingesetzt werden. Es ist auch möglich, das Aminierungsprodukt durch Destillation vom Katalysator zu trennen.

Wird die Reaktion in einem Lösungsmittels durchgeführt, so kann dieses mit dem Aminierungsprodukt mischbar sein und nach der Reaktion durch Destillation abgetrennt werden. Es können auch Lösungsmittel eingesetzt werden, welche eine Mischungslücke mit den Aminierungsprodukten oder den Edukten aufweisen. Als geeignete Lösungsmittel hierfür seien beispielsweise Toluol, Benzol, Xylole, Alkane, wie Hexane, Heptane und Oktane, und azyklische oder zyklische Ether, wie Diethylether, Tetrahydrofuran, Dioxan und tert-Amylalkohol genannt. Durch geeignete Wahl der Phosphinliganden löst sich der Katalysator bevorzugt in der Lösungsmittelphase, d.h. in der nicht-Produkt-enthaltenden Phase. Die Phosphinliganden können auch so gewählt werden, dass sich der Katalysator im Aminierungsprodukt löst. In diesem Fall lässt sich das Aminierungsprodukt durch Destillation vom Katalysator trennen.

Als Lösungsmittel kann auch das Produkt verwendet werden. Das Lösungsmittel kann auch unter den Reaktionsbedingungen mit den Edukten und dem Produkt mischbar sein und erst nach dem Abkühlen eine zweite flüssige Phase ausbilden, welche den Großteil des Katalysators enthält. Als Lösungsmittel, die diese Eigenschaft zeigen, seien beispielsweise Toluol, Benzol, Xylole, Alkane, wie Hexane, Heptane und Oktane, genannt. Der Katalysator kann dann zusammen mit dem Lösungsmittel abgetrennt und wieder verwendet werden. Die Produktphase kann auch in dieser Variante mit Wasser versetzt werden. Der in dem Produkt enthaltene Teil des Katalysators kann anschließend durch geeignete Absorbermaterialien wie beispielsweise Polyacrylsäure und deren Salze, sulfonierte Polystyrole und deren Salze, Aktivkohlen, Montmorillonite, Bentonite sowie Zeolithe abgetrennt werden oder aber in dem Produkt belassen werden.

Die Aminierungsreaktion kann auch zweiphasig durchgeführt werden. Bei der Ausführungsform der zweiphasigen Reaktionsführung eignen sich als unpolare Lösungsmittel besonders Toluol, Benzol, Xylole, Alkane, wie Hexane, Heptane und Oktane, in Kombination mit lipophilen Phosphinliganden am Übergangsmetallkatalysator, wodurch sich der Übergangsmetallkatalysator in der unpolaren Phase anreichert. Bei dieser Ausführungsform, in welcher das Produkt sowie das Reaktionswasser und ggf. nicht umgesetzte Edukte eine mit diesen Verbindungen angereicherte Zweitphase bilden, kann der Großteil des Katalysators durch einfache Phasentrennung von der Produktphase abgetrennt und wiederverwendet werden.

Falls flüchtige Nebenprodukte oder nicht umgesetzte Edukte oder auch das bei der Reaktion entstandene oder nach der Reaktion zur besseren Extraktion zugesetzte Wasser unerwünscht sind, können diese problemlos von dem Produkt durch Destillation abgetrennt werden.

Es kann auch vorteilhaft sein, das bei der Reaktion gebildete Wasser kontinuierlich aus dem Reaktionsgemisch zu entfernen. Das Reaktionswasser kann direkt durch Destillation aus dem Reaktionsgemisch oder als Azeotrop unter Zusatz eines geeigneten Lösungsmittels (Schleppers) und unter Verwendung eines Wasserabscheiders abgetrennt, oder durch Zusatz von Wasser entziehenden Hilfsstoffen entfernt werden.

Der Zusatz von Basen kann einen positiven Effekt auf die Produktbildung haben. Als geeignete Basen seien hier Alkalihydroxide, Erdalkalihydroxide, Alkalialkoholate, Erdalkalialkoholate, Alkali-Carbonate und Erdalkalicarbonate genannt, von welchen 0,01 bis 100 molare Äquivalente in Bezug auf den verwendeten Metallkatalysator eingesetzt werden können.

Ein weiterer Gegenstand der vorliegenden Erfindung ist die Verwendung eines Komplexkatalysators, der mindestens ein Element ausgewählt aus den Gruppen 8, 9 und 10 des Periodensystems sowie mindestens einen Donorliganden enthält, zur homogen-katalysierten Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak.

In einer bevorzugten Ausführungsform betrifft die vorliegende Erfindung die Verwendung eines homogen gelösten Komplexkatalysators der allgemeinen Formel (I) : 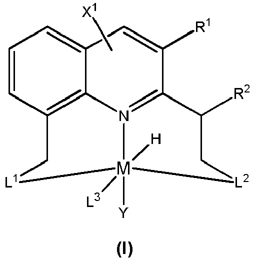 in der
- L¹ und L²: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterozyklische Carbene der Formel (II) oder (III): 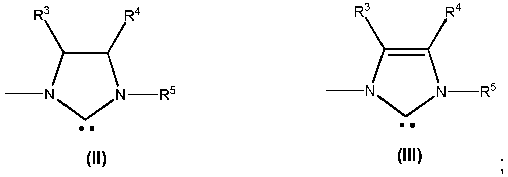
- L³: einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterozyklische Carbene der Formel II oder III;
- R¹ und R²: beide Wasserstoff oder gemeinsam mit den Kohlenstoffatomen, an die sie gebunden sind, ein Phenylring, der zusammen mit der Chinolinyleinheit der Formel I eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten, zur homogen-katalysierten Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak, wobei für den Katalysator der allgemeinen Formel I die vorstehend für das erfindungsgemäße Verfahren beschriebenen Definitionen und Bevorzugungen gelten.

In einer weiteren bevorzugten Ausführungsform betrifft die vorliegende Erfindung die Verwendung eines homogen gelösten Komplexkatalysators der allgemeinen Formel (XV): 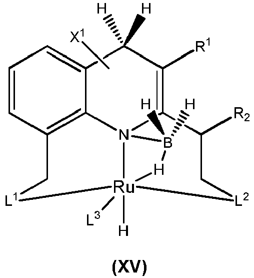 in der
- L¹ und L²: unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterozyklische Carbene der Formel (II) oder (III): 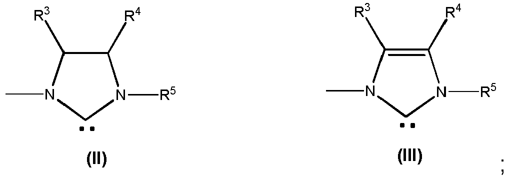
- L³: einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterozyklische Carbene der Formel (II) oder (III);
- R¹ und R²: beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
- R, R^{a}, R^{b}, R^{c}, R³, R⁴, und R⁵: unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
- Y: monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
- X¹: ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
- M: Eisen, Cobalt, Nickel, Ruthenium, Rhodium, Palladium, Osmium, Iridium oder Platin
bedeuten, zur homogen-katalysierten Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak, wobei für den Katalysator der allgemeinen Formel I die vorstehend für das erfindungsgemäße Verfahren beschriebenen Definitionen und Bevorzugungen gelten.

Die Erfindung wird durch die nachfolgenden Beispiele verdeutlicht, ohne sie darauf zu beschränken.

### Beispiele

### Generelle Vorschrift für die erfindungsgemäße katalytische Aminierung von Alkoholen mit Ammoniak

Katalysatorkomplex XIVb (Herstellung, siehe unten, Einwaage unter inerter Atmosphäre), Lösungsmittel (so viel, dass das Gesamtlösungsmittelvolumen 50 ml beträgt) und der umzusetzende Alkohol wurden unter Argon-Atmosphäre in einem 160 ml Parr-Autoklaven (Edelstahl V4A) mit magnetisch gekoppeltem Schrägblattrührer (Rührgeschwindigkeit: 200-500 Umdrehungen/Minute) vorgelegt. Die angegebene Menge an Ammoniak wurde bei Raumtemperatur entweder vorkondensiert oder direkt aus der NH₃-Druckgasflasche zudosiert. Falls Wasserstoff eingesetzt wurde, erfolgte dies durch iterative Differenzdruck-Dosierung. Der Stahlautoklav wurde auf die angegebene Temperatur elektrisch aufgeheizt und für die angegebene Zeit unter Rühren (500 Umdrehungen/Minute) erhitzt (Innentemperaturmessung). Nach dem Abkühlen auf Raumtemperatur, Entspannung des Autoklaven und Ausgasen des Ammoniaks bei Normaldruck wurde die Reaktionsmischung mittels GC (30m RTX5 Amin 0,32mm 1,5µm) analysiert. Das gewünschte Produkt lässt sich beispielsweise durch destillative Aufarbeitung isolieren. Die Ergebnisse für die Aminierung von 1,4-Butandiol (Tabelle 1), Diethylenglykol (Tabelle 2) und Monoethylenglykol (Tabelle 3), 2,5-Furandimethanol (Tabelle 4), Alkyldiolen (Tabelle 5), 1,4-Bis(hydroxymethyl)cyclohexan (Tabelle 6), Aminoalkoholen (Tabelle 7) sind im Folgenden angegeben.

### Synthese des Katalysatorkomplexes XIVb

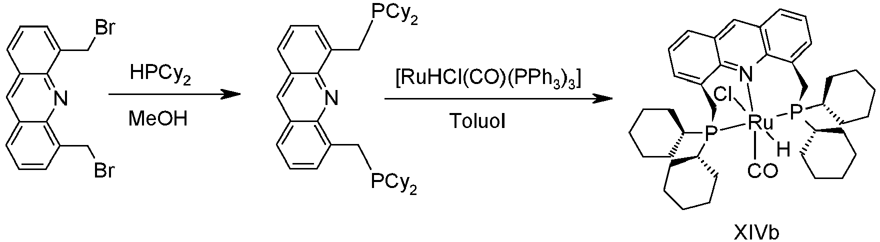

### a) Synthese von 4,5-Bis(dicyclohexylphosphinomethyl)acridin

Eine Lösung aus 4,5-Bis(bromomethyl)acridin¹ (5,2 g, 14,2 mmol) und Dicyclohexylphosphin (8,18 g, 36,8 mmol) in 65 mL wasserfreiem, entgasten Methanol wurde 66h auf 50°C unter einer inerten Argon-Atmosphäre erhitzt. Nach Abkühlen auf Raumtemperatur wurde Triethylamin (5,72 g, 56,7 mmol) zugegeben und 1 h gerührt. Verdampfen des Lösungsmittels ergab einen gelb-weißen Feststoff in rotem Öl. Extraktion mittels 3 x 40 mL MTBE und Konzentration des Filtrats ergaben ein rotbraunes Öl (¹H NMR: Mischung aus Produkt & HPCy₂). Aufnehmen in wenig warmen MTBE gefolgt von Zugabe von eisgekühltem Methanol resultierte in Ausfällung eines gelben, mikrokristallinen Feststoffes. Isolierung und Trocknung im Vakuum ergab luftempfindliches 4,5-Bis(dicyclohexylphosphinomethyl)acridin (2,74 g, 33%) als gelbes Pulver. ¹H NMR (360,63 MHz, Toluol-d8): δ [ppm] = 8,07 (s, 1 H, H9), 7,91 (d, *J =* 8,3 Hz, 2H, Ar-H), 7,42 (d, *J* = 8,3 Hz, 2H, Ar-H), 7,21 (dd, *J* = 8,3 Hz, *J* = 7,2 Hz, 2H, Ar-H), 3,89 (bs, 4H, -C*H*₂-P), 1,96-1,85 (m, 8H, Cy-H), 1,77-1,54 (m, 20H, Cy-H), 1,26-1,07 (m, 16H, Cy-H). ³¹P{¹H} NMR (145,98 MHz, Toluol-d8): δ [ppm] = 2,49 (s, -CH₂-*P*(Cy)₂).

### b) Synthese des Katalysatorkomplexes XIVb

4,5-Bis(dicyclohexylphosphinomethyl)acridin (1855 mg, 3,1 mmol) und [RuHCl(CO)(PPh₃)₃]² (2678 mg, 2,81 mmol) wurden für 2h in 80 mL entgastem Toluol auf 70°C erhitzt. Die resultierende dunkelbraune Lösung wurde zur Trockene eingeengt, der Rückstand in 3 x 20 mL Hexan aufgeschlämmt und durch Filtration isoliert. Trocknen im Vakuum ergab den Katalysatorkomplex XIVb (1603 mg, 75%) als orangebraunes Pulver. ¹H NMR (360,63 MHz, Toluol-d8): δ [ppm] = 8,06 (s, 1 H, H9), 7,43 (d, *J*= 7,6 Hz, 2H, Ar-H), 7,33 (d, *J* = 6,5 Hz, 2H, Ar-H), 7,06-7,02 (m, 2H, Ar-H), 5,02 (d, *J* = 11,9 Hz, 2H, -CH*H*-PCy₂), 3,54 (d, *J* = 12,2 Hz, 2H, -C*H*H-PCy₂), 2,87 (bs, 2H, - P(Cₐ*H*(CH₂)₅)₂), 2,54 (bs, 2H, -P(C_{b}*H*(CH₂)₅)₂), 2,18 (bs, 2H, Cy-H), 1,88-1,85 (m, 8H, Cy-H), 1,65 (bs, 6H, Cy-H), 1,42-1,35 (m, 14H, Cy-H), 1,17-0,82 (m, 12H, Cy-H), - 16,29 (t, *J* = 19,1 Hz, 1H, Ru-*H*). ³¹P{¹H} NMR (145,98 MHz, Toluol-d8): δ [ppm] = 60,89 (s, -CH₂-P(C_{y})₂).
[1] J. Chiron, J.P. Galy, Synlett, 2003, 15.
[2] Literaturvorschrift: Inorganic Syntheses 1974, 15, 48. Siehe auch: T. Joseph, S. S. Deshpande, S. B. Halligudi, A. Vinu, S. Ernst, M. Hartmann, J. Mol. Cat. (A) 2003, 206, 13-21.

**Tabelle 1a: Umsetzung von 1,4-Butandiol**

| | | | | | | | | | | |
|---|---|---|---|---|---|---|---|---|---|---|
| 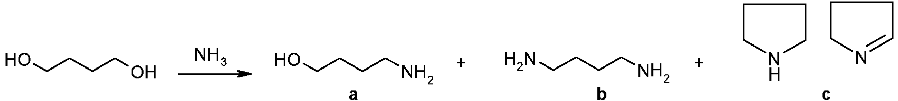 | | | | | | | | | | |

| **Nr^{a)}** | **Lösungsmittel** | **T [°C]** | **Zeit [h]** | **NH₃ [Eq]^{d)}** | **Reaktionsdruck [bar]** | **Weitere Bedingung** | **Umsatz^{b)}** | **Selektivität^{c)}** | | |
|---|---|---|---|---|---|---|---|---|---|---|
| | | | | | | | | **a** | **b** | **c** |
| 1 | Toluol | 155 | 12 | 6 | 44 | 0,2 mol% KOtBu | 43,3 | 60,1 | 12,1 | 18,7 |
| 2 | Toluol | 155 | 12 | 6 | 41 | 1,0 mol% KOtBu | 37,0 | 61,9 | 11,4 | 18,7 |
| 3 | Toluol | 155 | 24 | 9 | 51 | | 87,0 | 50,3 | 14,8 | 30,8 |
| 4 | Toluol | 155 | 60 | 6 | 57 | 5 bar H₂ kalt aufgepresst | 58,7 | 62,2 | 18,8 | 18,3 |
| 5 | p-Xylol | 180 | 12 | 6 | 51 | - | 100,0 | 0,6 | 51,0 | 43,6 |
| 6 | p-Xylol | 180 | 12 | 6 | 47 | 5,0 mol% Wasser | 99,9 | 0,7 | 46,7 | 48,6 |
| a) Bedingungen wenn nicht anders angegeben: 50 ml Lösungsmittel, Ansatzgröße 25 mmol 1,4-Butandiol, 0.1 mol% Katalysatorkomplex XIVb (pro Alkoholgruppe), b) Auswertung per GC (FI.%), c) Produktselektivität bestimmt per GC, d) Molare Äquivalente NH₃ pro OH-Funktion am Substrat; | | | | | | | | | | |

**Tabelle 1b: Umsetzung von 1,4-Butandiol**

| | | | | | | | | | | |
|---|---|---|---|---|---|---|---|---|---|---|
| 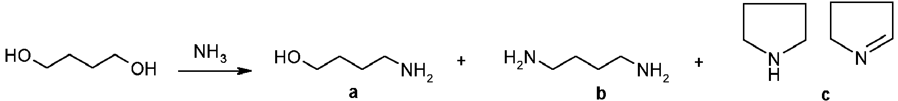 | | | | | | | | | | |

| **Nr**.**^{a)}** | **Lösungsmittel** | **Temp.** | **Zeit** | **NH₃** | **Reaktionsdruck (bar)** | **Weitere Bedingungen ^{e)}** | **Umsatz Edukt^{b)}** | **Selektivität^{c)}** | | |
|---|---|---|---|---|---|---|---|---|---|---|
| | **(wasserfrei)** | **[°C]** | **[h]** | **[eq]^{d)}** | | | | **a** | **b** | **c** |
| | | | | | | | **[%]** | **[%]** | **[%]** | **[%]** |
| 1 | Toluol | 155 | 12 | 6 | 46,1 | 0,2 mol% KOH (aq, 20%) | 59,25 | 59,14 | 16,42 | 19,89 |
| 2 | Toluol | 155 | 15 | 6 | 42,0 | -- | 96,06 | 17,90 | 14,20 | 62,10 |
| 3 | Toluol | 155 | 24 | 6 | 40,2 | -- | 98,92 | 8,60 | 20,38 | 64,91 |
| 4 | Toluol | 180 | 2 | 6 | 52,7 | -- | 91,52 | 36,35 | 25,76 | 35,39 |
| 5 | Toluol | 180 | 9 | 6 | 48,0 | -- | 100,00 | 0,12 | 19,90 | 73,67 |
| 6 | Toluol | 180 | 12 | 6 | 69,7 | 5 bar H2 | 94,19 | 30,09 | 37,23 | 31,62 |
| 7 | Toluol | 180 | 12 | 6 | 81,9 | 10 bar H2 | 89,85 | 36,24 | 35,41 | 27,66 |
| 8 | Dioxan | 180 | 12 | 6 | 44,3 | -- | 100,00 | 1,15 | 23,79 | 71,23 |
| 9 | THF | 180 | 12 | 6 | 46,9 | -- | 100,00 | 0,00 | 17,03 | 77,33 |
| 10 | THF | 180 | 12 | 9 | 62,3 | -- | 100,00 | 0,00 | 20,16 | 71,30 |
| 11 | THF | 180 | 12 | 6 | 71,7 | 5 bar H2 | 99,87 | 2,41 | 26,28 | 67,55 |
| a) Bedingungen wenn nicht anders angegeben: 50 ml Lösungsmittel, Ansatzgröße 25 mmol 1,4-Butandiol, b) Auswertung per GC (FI.%), c) Produktselektivität bestimmt per GC, d) Molare Äquivalente NH3 pro OH-Funktion am Substrat, e) Bezug der mol%-Angaben pro Alkoholgruppe | | | | | | | | | | |

**Tabelle 2a: Umsetzung von Diethylenglykol**

| | | | | | | | | | | |
|---|---|---|---|---|---|---|---|---|---|---|
| 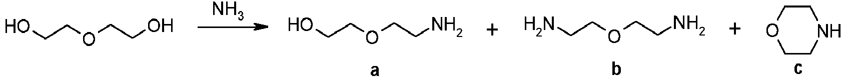 | | | | | | | | | | |

| **Nr^{a)}** | **Lösungsmittel** | **T [°C]** | **Zeit [h]** | **NH₃ [Eq]^{d)}** | **Reaktionsdruck [bar]** | **Weitere Bedingungen** | **Umsatz^{b)}** | **Selektivität^{c)}** | | |
|---|---|---|---|---|---|---|---|---|---|---|
| | | | | | | | | **a** | **b** | **c** |
| 1 | Toluol | 155 | 12 | 6 | 40 | | 79,0 | 51,4 | 23,8 | 12,9 |
| 2 | Toluol | 155 | 12 | 6 | 43 | | 82,4 | 55,3 | 20,1 | 10,9 |
| 3 | Toluol | 155 | 12 | 6 | 42 | 0,2 mol% KOtBu | 69,8 | 41,8 | 31,9 | 14,3 |
| 4 | Toluol | 155 | 12 | 6 | 43 | 1,0 mol% KOtBu | 60,4 | 44,7 | 25,8 | 14,8 |
| 5^{f)} | Toluol | 155 | 60 | 6 | 58 | 5 bar H2 | 66,5 | 57,1 | 31,0 | 9,9 |
| 6 | p-Xylol | 155 | 12 | 6 | 38 | 1,0 mol% Wasser | 77,5 | 52,9 | 21,6 | 16,9 |
| 7 | p-Xylol | 155 | 12 | 6 | 41 | 5 mol% Wasser | 84,0 | 49,0 | 21,1 | 12,8 |
| 8 | p-Xylol | 155 | 15 | 6 | 46 | | 77,5 | 49,1 | 23,7 | 13,1 |
| 9 | p-Xylol | 155 | 24 | 6 | 44 | | 96,3 | 17,0 | 48,6 | 19,9 |
| 10 | p-Xylol | 155 | 24 | 6 | 53 | 1,0 mol% Wasser | 84,6 | 51,8 | 20,8 | 12,9 |
| 11 | p-Xylol | 180 | 12 | 6 | 50 | | 100,0 | 0,4 | 46,1 | 27,9 |
| 12 | p-Xylol | 180 | 12 | 6 | 50 | 5mol% H2O | 100,0 | 0,4 | 48,2 | 27,4 |
| a) Bedingungen wenn nicht anders angegeben: 50 ml Lösungsmittel, Ansatzgröße 25 mmol Diethylenglykol, 0.1 mol% Katalysatorkomplex XIVb (pro Alkoholgruppe), b) Auswertung per GC (FI.%), c) Produktselektivität bestimmt per GC, d) Molare Äquivalente NH₃ pro OH-Funktion am Substrat; f) Ansatzgröße 35 mmol Diethylenglykol in 70 ml Lösungsmittel | | | | | | | | | | |

**Tabelle 2b: Umsetzung von Diethylenglykol**

| | | | | | | | | | | | |
|---|---|---|---|---|---|---|---|---|---|---|---|
| 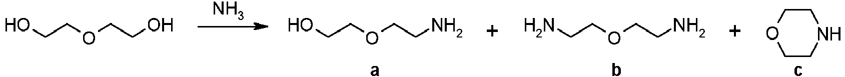 | | | | | | | | | | | |

| **Nr**.^{**a**)} | **Lösungsmittel (wasserfrei)** | **Katalysator** | **Temp. [°C]** | **Zeit [h]** | **NH₃ [eq]^{d)}** | **Reaktionsdruck (bar)** | **Weitere Bedingungen ^{e)}** | **Umsatz Edukt^{b)}** | | **Selektivität^{c)}** | |
|---|---|---|---|---|---|---|---|---|---|---|---|
| | | | | | | | | | **a** | **b** | **c** |
| | | | | | | | | **[%]** | **[%]** | **[%]** | **[%]** |
| 1 | Toluol | XIVb | 155 | 12 | 2,0 | 12,3 | | 83,52 | 37,60 | 13,93 | 25,58 |
| 2 | Toluol | XIVb | 155 | 12 | 6 | 40,9 | 0,2 mol% KOH (aq, 20%) | 73,94 | 39,35 | 37,29 | 15,28 |
| 3 | Toluol | XIVb | 155 | 24 | 6 | 43,6 | | 97,31 | 18,10 | 36,58 | 21,66 |
| 4 | Toluol | XIVb | 155 | 15 | 6 | 45,7 | 0.05 mol% XIVb | 95,97 | 17,66 | 40,46 | 26,67 |
| 5 | Toluol | XIVb | 155 | 12 | 6 | 65,5 | 5 bar H2 | 61,84 | 69,16 | 18,61 | 8,01 |
| 6 | Toluol | XIVb | 155 | 12 | 6 | 36,0 | 25 g t-Butanol, 25 ml Toluol | 86,90 | 44,98 | 26,76 | 15,52 |
| 7 | Toluol | XIVb | 165 | 12 | 6 | 45,1 | | 98,22 | 12,52 | 40,92 | 21,86 |
| 8 | Toluol | XIVb | 170 | 12 | 6 | 45,7 | | 99,81 | 4,39 | 43,66 | 26,02 |
| 9 | Toluol | XIVb | 180 | 2 | 6 | 47,2 | 0,2 mol% XIVb | 95,81 | 19,45 | 41,17 | 19,87 |
| 10 | Toluol | XIVb | 180 | 9 | 6 | 45,5 | | 100,00 | 0,75 | 39,21 | 29,46 |
| 11 | Toluol | XIVb | 180 | 12 | 6 | 37,7 | | 100,00 | 0,00 | 32,75 | 38,67 |
| 12 | Toluol | XIVb | 180 | 12 | 6 | 69,7 | 5 bar H2 | 96,05 | 20,68 | 54,70 | 16,64 |
| 13 | Toluol | XIVb | 180 | 12 | 6 | 75,6 | 10 bar H2 | 86,11 | 35,73 | 47,22 | 13,77 |
| 14 | Dioxan | XIVb | 155 | 12 | 6 | 38,0 | | 68,17 | 65,02 | 20,29 | 9,21 |
| 15 | Dioxan | XIVb | 180 | 12 | 6 | 34,1 | | 99,66 | 4,65 | 40,23 | 34,65 |
| 16 | THF | XIVb | 155 | 12 | 6 | 41,0 | | 70,97 | 54,46 | 19,41 | 11,95 |
| 17 | THF | XIVb | 155 | 12 | 9 | 51,9 | | 81,65 | 53,75 | 23,60 | 13,51 |
| 18 | THF | XIVb | 180 | 12 | 6 | 49,1 | | 100,00 | 0,00 | 42,48 | 41,98 |
| 19 | Toluol | XIVa | 155 | 12 | 6 | 40,7 | | 68,02 | 69,62 | 9,60 | 9,52 |
| 20 | Toluol | XIVa | 155 | 24 | 6 | 42,1 | | 77,16 | 43,54 | 20,09 | 15,10 |
| a) Bedingungen wenn nicht anders angegeben: 50 ml Lösungsmittel, Ansatzgröße 25 mmol Diethylenglykol, 0,1 mol% Katalysatorkomplex XIVa oderXIVb (pro Alkoholgruppe), b) Auswertung per GC (FI.%), c) Produktselektivität bestimmt per GC, d) Molare Äquivalente NH3 pro OH-Funktion am Substrat, e) Bezug der mol%-Angaben pro Alkoholgruppe, | | | | | | | | | | | |

**Tabelle 3a: Umsetzung von MEG (Monoethylenglykol)**

| | | | | | | | | | | |
|---|---|---|---|---|---|---|---|---|---|---|
| 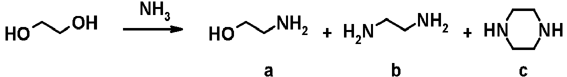 | | | | | | | | | | |

| **Nr^{a)}** | **Lösungsmittel** | **T [°C]** | **Zeit [h]** | **NH₃ [Eq.]^{d)}** | **Reaktionsdruck [bar]** | **Weitere Bedingungen** | **Umsatz^{b)}** | **Selektivität^{c)}** | | |
|---|---|---|---|---|---|---|---|---|---|---|
| | | | | | | | | **a** | **b** | **c** |
| 1 | Toluol | 155 | 12 | 6 | 42 | 0.2 mol% KOtBu | 62,9 | 47,5 | 25,0 | 0,5 |
| 2 | Toluol | 155 | 12 | 6 | 41 | 1 mol% KOtBu | 75,9 | 39,9 | 26,8 | 0,3 |
| 3 | Toluol | 155 | 12 | 6 | 44 | | 19,3 | 48,3 | 21,8 | 0,6 |
| 4 | Toluol | 155 | 12 | 6 | 42 | 17 Eq. Wasser | 21,6 | 55,6 | 36,4 | 0,0 |
| a) Bedingungen wenn nicht anders angegeben: 50 ml Lösungsmittel, Ansatzgröße 25 mmol Monoethylenglykol, 0.1 mol% Katalysatorkomplex XIVb (pro Alkoholgruppe), b) Auswertung per GC (FI.%), c) Produktselektivität bestimmt per GC, d) Molare Äquivalente NH₃ pro OH-Funktion am Substrat; | | | | | | | | | | |

**Tabelle 3b: Umsetzung von MEG (Monoethylenglykol)**

| | | | | | | | | | | | |
|---|---|---|---|---|---|---|---|---|---|---|---|
| 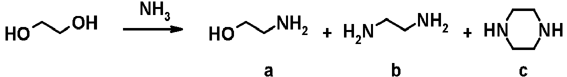 | | | | | | | | | | | |

| **Nr.^{a)}** | **Lösungsmittel (wasserfrei)** | **Katalysator** | **Temp. [°C]** | **Zeit [h]** | **NH₃ [eq]^{d)}** | **Reaktionsdruck (bar)** | **Weitere Bedingungen ^{e)}** | **Umsatz Edukt^{b)}** | **Selektivität^{c)}** | | |
|---|---|---|---|---|---|---|---|---|---|---|---|
| | | | | | | | | | **a** | **b** | **c** |
| | | | | | | | | **[%]** | **[%]** | **[%]** | **[%]** |
| 1 | Toluol | XIVb | 155 | 12 | 6 | 39,8 | 0,2 mol% KOH (aq, 20%) | 59,98 | 41,02 | 22,73 | 12,92 |
| 2 | Toluol | XIVb | 180 | 12 | 6 | 46,8 | -- | 94,72 | 11,00 | 19,72 | 44,48 |
| 3 | Toluol | XIVb | 180 | 12 | 6 | 47,4 | 1 mol% KOtBu | 100,00 | 0,66 | 21,17 | 49,04 |
| 4 | Toluol | XIVb | 180 | 12 | 6 | 66,1 | 5 bar H2 | 85,23 | 15,49 | 26,30 | 45,17 |
| 5 | p-Xylol | XIVb | 155 | 24 | 6 | 45,8 | -- | 45,78 | 43,94 | 18,28 | 0,22 |
| 6 | THF | XIVb | 155 | 12 | 6 | 41,7 | 2 mol% KOtBu | 56,85 | 47,52 | 18,66 | 1,98 |
| 7 | THF | XIVb | 180 | 12 | 6 | 47,2 | -- | 88,49 | 10,02 | 22,50 | 46,63 |
| | | | | | | | | | | | |
| 8 | Toluol | XIVa | 180 | 24 | 6 | 28,0 | -- | 100,00 | 6,39 | 11,51 | 60,53 |
| 9 | Toluol | XIVa | 155 | 12 | 6 | 40,8 | 1 mol% KOtBu | 50,47 | 52,84 | 19,81 | 4,31 |
| a) Bedingungen wenn nicht anders angegeben: 50 ml Lösungsmittel, Ansatzgröße 25 mmol Monoethylenglykol, 0,1 mol% Katalysatorkomplex XIVa oder XIVb (pro Alkoholgruppe), b) Auswertung per GC (FI.%), c) Produktselektivität bestimmt per GC, d) Molare Äquivalente NH3 pro OH-Funktion am Substrat, e) Bezug der mol%-Angaben pro Alkoholgruppe | | | | | | | | | | | |

**Tabelle 4: Umsetzung von 2,5-Furandimethanol:**

| | | | | | | | | | |
|---|---|---|---|---|---|---|---|---|---|
| 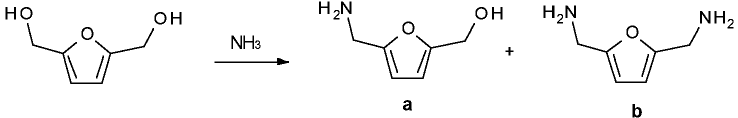 | | | | | | | | | |

| | **Lösungsmittel** | **Katalysator** | **Temp.** | **Zeit [h]** | **NH₃** | **Reaktionsdruck** | **Umsatz Edukt^{b)}** | **Selektivität ^{c)}** | |
|---|---|---|---|---|---|---|---|---|---|
| **Nr.^{a)}** | **(wasserfrei)** | | **[°C]** | | **[eq]^{d)}** | **(bar)** | | **a** | **b** |
| | | | | | | | **[%]** | **[%]** | **[%]** |
| 1 | THF | XIVb | 140 | 21 | 6 | 35,2 | 100,00 | 0,40 | 96,36 |
| 2 | THF | XIVb | 150 | 6 | 6 | 38,8 | 100,00 | 7,14 | 87,75 |
| 3 | THF | XIVb | 150 | 12 | 6 | 40,4 | 100,00 | 0,27 | 84,44 |
| 4 | THF | XIVb | 150 | 18 | 6 | 37,1 | 100,00 | 0,31 | 94,15 |
| 5 | t-Amylalkohol | XIVb | 140 | 9 | 6 | 31,4 | 99,59 | 9,55 | 84,97 |
| 6 | t-Amylalkohol | XIVb | 150 | 5 | 6 | 37,1 | 100,00 | 2,70 | 90,10 |
| 7 | t-Amylalkohol | XIVb | 150 | 18 | 6 | 37,9 | 100,00 | 0,00 | 95,60 |
| a) Bedingungen wenn nicht anders angegeben: 50 ml Lösungsmittel, Ansatzgröße 25 mmol 2,5-Furandimethanol, 0,1 mol% Katalysatorkomplex XIVB (pro Alkoholgruppe), b) Auswertung per GC (FI.%), c) Produktselektivität bestimmt per GC, d) Molare Äquivalente NH3 pro OH-Funktion am Substrat | | | | | | | | | |

**Tabelle 5: Umsetzung von Alkyldiolen**

| | | | | | | | | | | | |
|---|---|---|---|---|---|---|---|---|---|---|---|
| 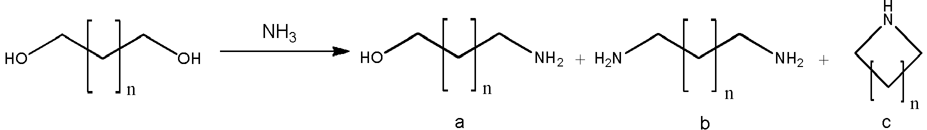 | | | | | | | | | | | |
| n=1 bis 34 | | | | | | | | | | | |

| **Nr**.^{**a**)} | **Alkohol** | **Lösungsmittel (wasserfrei)** | **Katalysator** | **Temp. [°C]** | **Zeit [h]** | **NH₃ [eq]^{d)}** | **Reaktionsdruck (bar)** | **Umsatz Edukt^{b)}** | **Selektivität^{c)}** | | |
|---|---|---|---|---|---|---|---|---|---|---|---|
| | | | | | | | | | **a** | **b** | **c** |
| | | | | | | | | **[%]** | **[%]** | **[%]** | **[%]** |
| 1 | 1,3-Propandiol | Toluol | XIVb | 135 | 12 | 6 | 41,1 | 99,73 | 8,95 | 35,79 | |
| 2 | 1,5-Pentandiol | Toluol | XIVb | 180 | 12 | 6 | 44,1 | 80,51 | 58,26 | 19,24 | 15,13 |
| 3 | 1,6-Hexandiol | Toluol | XIVb | 155 | 12 | 6 | 34,0 | 100,00 | 1,14 | 91,38 | 0,51 |
| 4^{e)} | 1,9-Nonandiol | THF | XIVb | 150 | 24 | 6 | 15,0 | 97,70 | 10,60 | 74,60 | |
| 5 | 1,10-Decandiol | Toluol | XIVb | 155 | 12 | 6 | 44,3 | 95,19 | 1,36 | 93,25 | |
| 6 | C36-Diol | THF | XIVb | 155 | 12 | 6 | 38,2 | Aminzahl (AZ) ^{f)}: 197 | | | |
| | | | | | | | | AZ (primäre Amine): 196 | | | |
| | | | | | | | | AZ (sekundäre Amine) <1 | | | |
| | | | | | | | | AZ (tertiäre Amine): 1 | | | |
| a) Bedingungen wenn nicht anders angegeben: 50 ml Lösungsmittel, Ansatzgröße: 25 mmol Alkyldiol, 0,1 mol% Katalysatorkomplex XIVb (pro Alkoholgruppe), b) Auswertung per GC (FI.%), c) Produktselektivität bestimmt per GC, d) Molare Äquivalente NH3 pro OH-Funktion am Substrat, e) Ansatzgröße: 50 mmol 1,9-Nonandiol im 100 mL-Autoklaven, f) Definition Aminzahl siehe Thieme Römpp Chemielexikon | | | | | | | | | | | |

**Tabelle 6: Umsetzung von 1,4-Bis(aminomethyl)cyclohexan**

| | | | | | | | | | | |
|---|---|---|---|---|---|---|---|---|---|---|
| 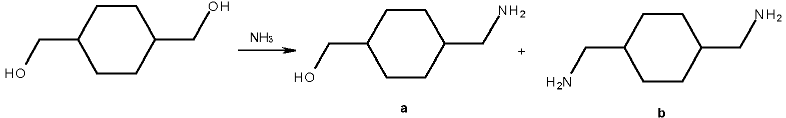 | | | | | | | | | | |

| **Nr**.**^{a)}** | **Alkohol** | **Lösungsmittel (wasserfrei)** | **Katalysator** | **Temp. [°C]** | **Zeit [h]** | **NH₃ [eq]^{d)}** | **Reaktionsdruck (bar)** | **Umsatz Edukt^{b)}** | **Selektivität ^{c)}** | |
|---|---|---|---|---|---|---|---|---|---|---|
| | | | | | | | | | **a** | **b** |
| | | | | | | | | **[%]** | **[%]** | **[%]** |
| 1 | 1,4-Bis(hydroxymethyl)cyclohexan | THF | XIVb | 155 | 12 | 6 | 45,5 | 100,00 | 0,63 | 94,35 |
| a) 50 ml Lösungsmittel, 25 mmol 1,4-(Bishydroxymethyl)cyclohexan, 0,1 mol% Katalysatorkomplex XIVb (pro Alkoholgruppe), | | | | | | | | | | |
| b) Auswertung per GC (FI.%), c) Produktselektivität bestimmt per GC, d) Molare Äquivalente NH3 pro OH-Funktion am Substrat | | | | | | | | | | |

**Tabelle 7: Umsetzung von α,ω-Alkanolaminen**

| | | | | | | | | | | |
|---|---|---|---|---|---|---|---|---|---|---|
| 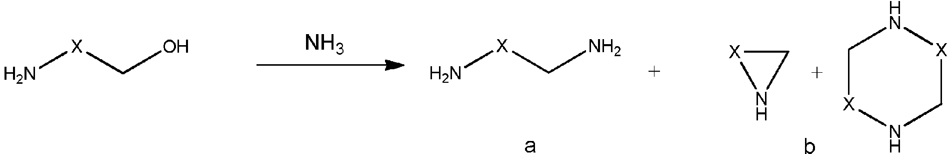 | | | | | | | | | | |
| X = (CH₂)₁₋₃, C₂H₄OCH₂ | | | | | | | | | | |

| **Nr**.**^{a)}** | **Alkohol** | **Lösungsmittel (wasserfrei)** | **Katalysator** | **Temp. [°C]** | **Zeit [h]** | **NH₃ [eq]^{d)}** | **Reaktionsdruck (bar)** | **Umsatz Edukt^{b)}** | **Selektivität^{c)}** | |
|---|---|---|---|---|---|---|---|---|---|---|
| | | | | | | | | | **a** | **b** |
| | | | | | | | | **[%]** | **[%]** | **[%]** |
| 1 | 3-Aminopropan-1-ol | Toluol | XIVb | 135 | 12 | 6 | 35,2 | 45,54 | 46,98 | |
| 2^{e)} | 4-Aminobutan-1-ol | THF | XIVb | 180 | 12 | 6 | 24,8 | 77,21 | 9,48 | 85,24 |
| 3 | 2-(2-Aminoethoxy)ethanol | Toluol | XIVb | 155 | 15 | 6 | 41,7 | 41,01 | 50,29 | 24,58 |
| 4 | Monoaminoethanol | Toluol | XIVb | 155 | 15 | 6 | 42,3 | 72,86 | 69,39 | 12,28 |
| 5 | Monoaminoethanol | Toluol | XIVb | 180 | 12 | 6 | 71,5 | 95,92 | 66,17 | 19,25 |
| a) Bedingungen wenn nicht anders angegeben: 50 ml Lösungsmittel, Ansatzgröße: 25 mmol Alkanolamin, 0,1 mol% Katalysatorkomplex XIVb (pro Alkoholgruppe), | | | | | | | | | | |
| b) Auswertung per GC (FI.%), c) Produktselektivität bestimmt per GC, d) Molare Äquivalente NH3 pro OH-Funktion am Substrat, e) Ansatzgröße: 50 mmol im 300 mL-Autoklaven. | | | | | | | | | | |

## Patentansprüche

1. Verfahren zur Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak unter Wasserabspaltung, wobei die Reaktion homogen-katalysiert in Gegenwart mindestens eines Komplexkatalysators, der mindestens ein Element ausgewählt aus den Gruppen 8, 9 und 10 des Periodensystems sowie mindestens einen Donorliganden enthält, durchgeführt wird, wobei der Komplexkatalysator ein Katalysator der Formel I ist: 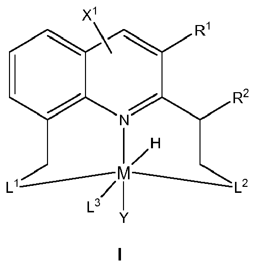 in der
L¹ und L² unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterocyclische Carbene der Formel II oder III: 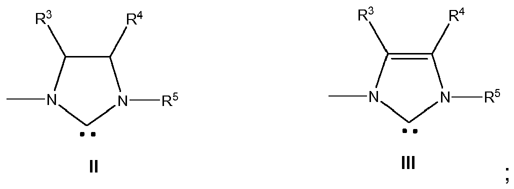
L³ einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterocyclische Carbene der Formel II oder III;
R¹ und R² beide Wasserstoff oder gemeinsam mit den Kohlenstoffatomen, an die sie gebunden sind, ein Phenylring, der zusammen mit der Chinolinyleinheit der Formel I eine Acridinyleinheit bildet;
R, R^{a}, R^{b}, R^{c}, R³, R⁴, und R⁵ unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
X¹ ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
M Eisen, Cobalt, Nickel, Ruthenium, Rhodium, Palladium, Osmium, Iridium oder Platin
bedeuten.

2. Verfahren nach Anspruch 1, wobei R¹ und R² beide Wasserstoff sind und der Komplexkatalysator ein Katalysator der Formel (IV) ist: 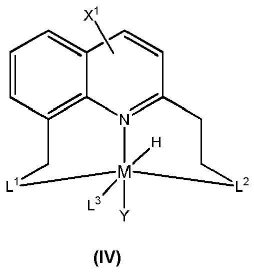 und X¹, L¹, L², L³ und Y die in Anspruch 1 genannten Bedeutungen aufweisen.

3. Verfahren nach Anspruch 1, wobei R¹ und R² gemeinsam mit den Kohlenstoffatomen, an die sie gebunden sind, ein Phenylring bilden, der zusammen mit der Chinolinyleinheit der Formel I eine Acridinyleinheit bildet und der Komplexkatalysator ein Katalysator der Formel (V) ist: 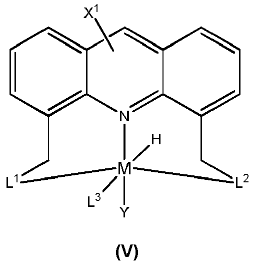 und X¹, L¹, L², L³ und Y die in Anspruch 1 genannten Bedeutungen aufweisen.

4. Verfahren nach Anspruch 1, wobei der Komplexkatalysator aus der Gruppe der Katalysatoren der Formel (VI), (VII), (VIII), (IX), (X), (XI), (XII) und (XIII) ausgewählt ist: 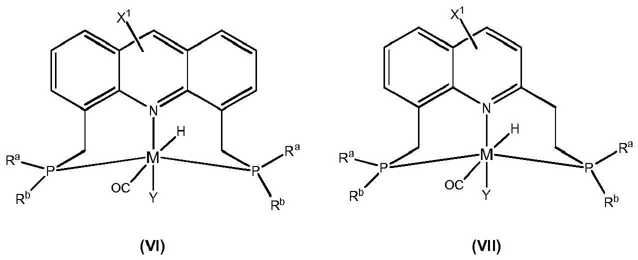 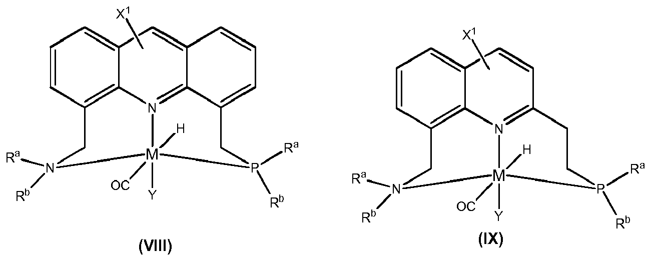 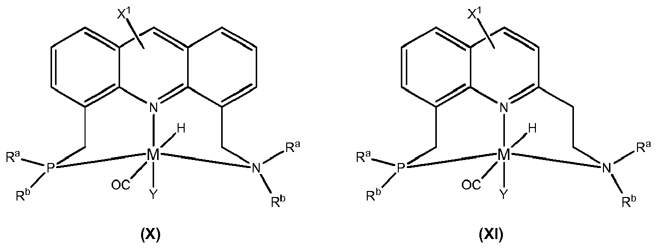 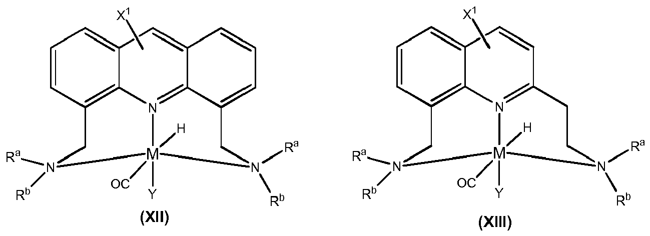 und X¹, R^{a}, R^{b}, und Y die in Anspruch 1 genannten Bedeutungen aufweisen.

5. Verfahren nach Anspruch 1, wobei der Komplexkatalysator ein Katalysator der Formel (XIVa) ist:

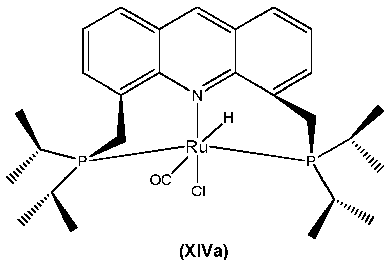

6. Verfahren nach Anspruch 1, wobei der Komplexkatalysator ein Katalysator der Formel (XIVb) ist:

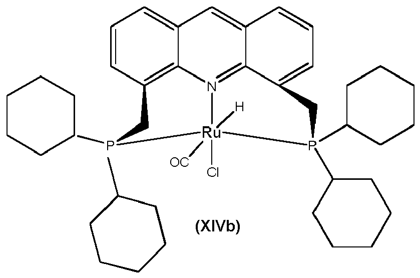

7. Verfahren nach Anspruch 1, wobei der Komplexkatalysator ein Katalysator der Formel (XV) ist: 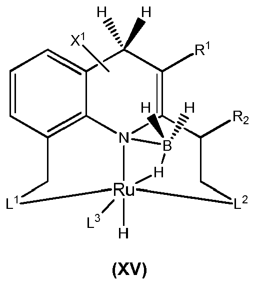 in der
L¹ und L² unabhängig voneinander PR^{a}R^{b}, NR^{a}R^{b}, Sulfid, SH, S(=O)R, C₅-C₁₀-Heteroaryl enthaltend mindestens ein Heteroatom ausgewählt aus N, O und S, AsR^{a}R^{b}, SbR^{a}R^{b} oder N-heterocyclische Carbene der Formel (II) oder (III): 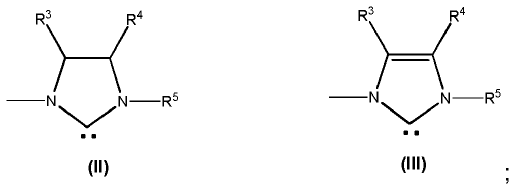
L³ einzähniger Zweielektronendonor ausgewählt aus der Gruppe CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, Pyridin, Thiophen, Tetrahydrothiophen und N-heterocyclische Carbene der Formel (II) oder (III);
R¹ und R² beide Wasserstoff oder gemeinsam mit den Kohlenstoffatomen, an die sie gebunden sind, ein Phenylring, der zusammen mit der Chinolinyleinheit der Formel (I) eine Acridinyleinheit bildet;
R, R^{a}, R^{b}, R^{c}, R³, R⁴, und R⁵ unabhängig voneinander unsubstituiertes oder zumindest monosubstituiertes C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl oder C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
Y monoanionischer Ligand ausgewählt aus der Gruppe H, F, Cl, Br, I, OCOR, OCOCF₃, OSO₂R, OSO₂CF₃, CN, OH, OR und N(R)₂ oder neutrale Moleküle ausgewählt aus der Gruppe NH₃, N(R)₃ und R₂NSO₂R;
X¹ ein, zwei, drei, vier, fünf, sechs, oder sieben Substituenten an einem oder mehreren Atomen der Acridinyleinheit oder ein, zwei, drei, vier oder fünf Substituenten an einem oder mehreren Atomen der Chinolinyleinheit,
wobei X¹ unabhängig voneinander ausgewählt ist aus der Gruppe bestehend aus Wasserstoff, F, Cl, Br, I, OH, NH₂, NO₂, -NC(O)R, C(O)NR₂, -OC(O)R, -C(O)OR, CN und Boranderivaten, die aus dem Katalysator der Formel I durch Reaktion mit NaBH₄ erhältlich sind und unsubstituiertem oder zumindest monosubstituiertem C₁-C₁₀-Alkoxy, C₁-C₁₀-Alkyl, C₃-C₁₀-Cycloalkyl, C₃-C₁₀-Heterocyclyl enthaltend mindestens ein Heteroatom ausgewählt aus N, O und S, C₅-C₁₀-Aryl und C₅-C₁₀-Heteroaryl enthaltend mindestens ein Heteroatom ausgewählt aus N, O und S,
wobei die Substituenten ausgewählt sind aus der Gruppe bestehend aus: F, Cl, Br, OH, CN, NH₂ und C₁-C₁₀-Alkyl;
und
M Eisen, Cobalt, Nickel, Ruthenium, Rhodium, Palladium, Osmium, Iridium oder Platin
bedeuten.

8. Verfahren nach einem der Ansprüche 1 oder 7, wobei der Komplexkatalysator ein Katalysator der Formel (XVIa) ist:

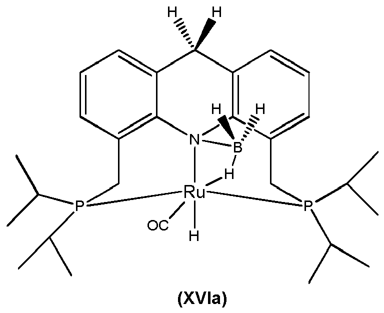

9. Verfahren nach einem der Ansprüche 1 bis 4, wobei im Komplexkatalysator Y ausgewählt ist aus H, F, Cl und Br.

10. Verfahren nach einem der Ansprüche 1 bis 9, wobei im Komplexkatalysator L³ CO ist.

11. Verfahren nach einem der Ansprüche 1 bis 10, wobei (-X) ausgewählt ist aus funktionellen Gruppen der Formeln (-CH₂-OH) und (-CH₂-NH₂).

12. Verfahren nach einem der Ansprüche 1 bis 11, wobei als Diol Diethylenglykol eingesetzt wird

13. Verfahren nach einem der Ansprüche 1 bis 11, wobei ein Diol ausgewählt aus der Gruppe Ethylenglykol, Diethanolamin, Polytetrahydrofuran und 1,4-Butandiol eingesetzt wird.

14. Verwendung eines Komplexkatalysators, der mindestens ein Element ausgewählt aus den Gruppen 8, 9 und 10 des Periodensystems sowie mindestens einen Phosphordonorliganden enthält, zur homogen-katalysierten Herstellung von primären Aminen, die mindestens eine funktionelle Gruppe der Formel (-CH₂-NH₂) und mindestens eine weitere primäre Aminogruppe aufweisen, durch Alkoholaminierung von Edukten, die mindestens eine funktionelle Gruppe der Formel (-CH₂-OH) und mindestens eine weitere funktionelle Gruppe (-X) aufweisen, wobei (-X) ausgewählt ist aus Hydroxylgruppen und primären Aminogruppen, mit Ammoniak.

## Claims

1. A process for preparing primary amines which have at least one functional group of the formula (-CH₂-NH₂) and at least one further primary amino group by alcohol amination of starting materials having at least one functional group of the formula (-CH₂-OH) and at least one further functional group (-X), where (-X) is selected from among hydroxyl groups and primary amino groups, by means of ammonia with elimination of water, wherein the reaction is carried out homogeneously catalyzed in the presence of at least one complex catalyst comprising at least one element selected from groups 8, 9 and 10 of the Periodic Table and also at least one donor ligand,
wherein the complex catalyst is a catalyst of the formula I: 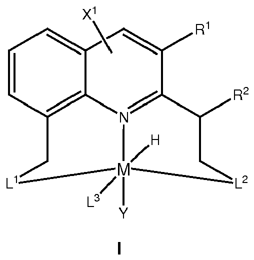 where
L¹ and L² are each, independently of one another, PR^{a}R^{b}, NR³R^{b}, sulfide, SH, S(=O)R, C₅-C₁₀-heteroaryl comprising at least one heteroatom selected from among N, 0 and S, AsR^{a}R^{b}, SbR^{a}R^{b} and N-heterocyclic carbenes of the formula II or III: 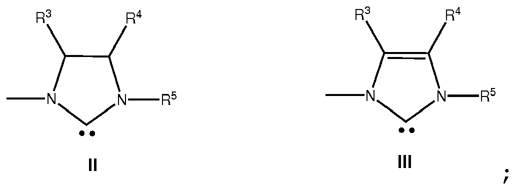
L³ is a monodentate two-electron donor selected from the group consisting of CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophene, tetrahydrothiophene and N-heterocyclic carbenes of the formula II or III;
R¹ and R² are both hydrogen or together with the carbon atoms to which they are bound form a phenyl ring which together with the quinolinyl unit of the formula I forms an acridinyl unit;
R, R^{a}, R^{b}, R^{c}, R³, R⁴ and R⁵ are each, independently of one another, unsubstituted or at least monosubstituted C₁-C₁₀-alkyl, C₃-C₁₀-cycloalkyl, C₃-C₁₀-heterocyclyl comprising at least one heteroatom selected from among N, 0 and S, C₅-C₁₀-aryl or C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
Y is a monoanionic ligand selected from the group consisting of H, F, Cl, Br, I, OCOR, OCOCF₃, OSO₂R, OSO₂CF₃, CN, OH, OR and N(R)₂ or an uncharged molecule selected from the group consisting of NH₃, N(R)₃ and R₂NSO₂R;
X¹ represents one, two, three, four, five, six or seven substituents on one or more atoms of the acridinyl unit or one, two, three, four or five substituents on one or more atoms of the quinolinyl unit,
where the radicals X¹ are selected independently from the group consisting of hydrogen, F, Cl, Br, I, OH, NH₂, NO₂,-NC(O)R, C(O)NR₂, -OC(O)R, -C(O)OR, CN and borane derivatives which can be obtained from the catalyst of the formula I by reaction with NaBH₄ and unsubstituted or at least monosubstituted C₁-C₁₀-alkoxy, C₁-C₁₀-alkyl, C₃-C₁₀-cycloalkyl, C₃-C₁₀-heterocyclyl comprising at least one heteroatom selected from among N, O and S, C₅-C₁₀-aryl and C₅-C₁₀-heteroaryl comprising at least one heteroatom selected from among N, O and S,
where the substitutents are selected from the group consisting of: F, Cl, Br, OH, CN, NH₂ and C₁-C₁₀-alkyl;
and
M is iron, cobalt, nickel, ruthenium, rhodium, palladium, osmium, iridium or platinum.

2. The process according to claim 1, wherein R¹ and R² are both hydrogen and the complex catalyst is a catalyst of the formula (IV): 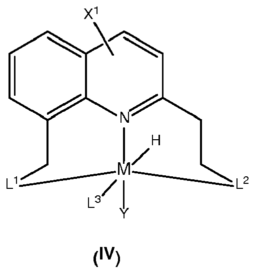 and X¹, L¹, L², L³ and Y are as defined in claim 1.

3. The process according to claim 1, wherein R¹ and R² together with the carbon atoms to which they are bound form a phenyl ring which together with the quinolinyl units of the formula I forms an acridinyl unit and the complex catalyst is a catalyst of the formula (V): 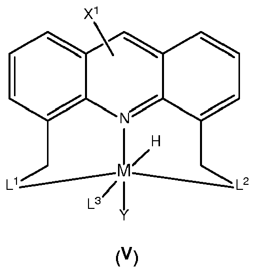 and X¹, L¹, L², L³ and Y are as defined in claim 1.

4. The process according to claim 1, wherein the complex catalyst is selected from the group of catalysts of the formulae (VI), (VII), (VIII), (IX), (X), (XI), (XII) and (XIII): 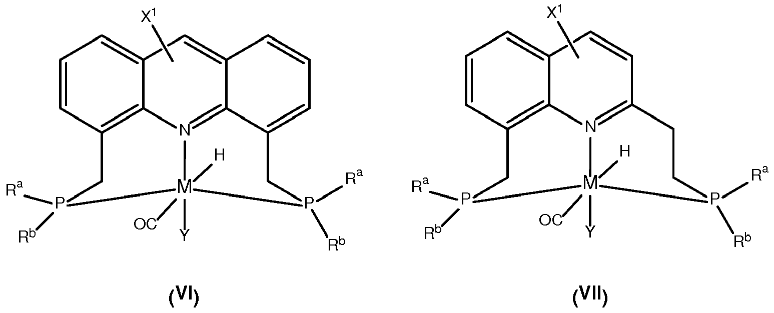 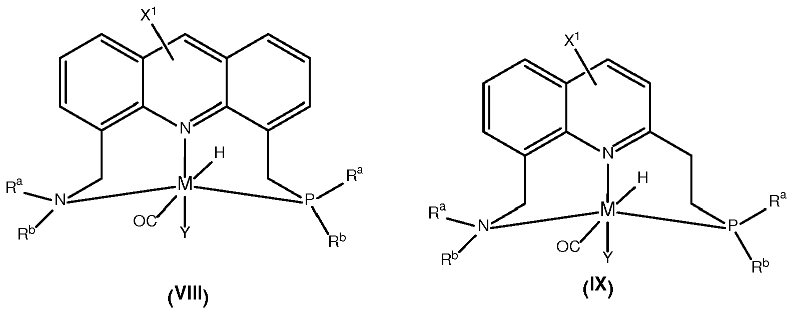 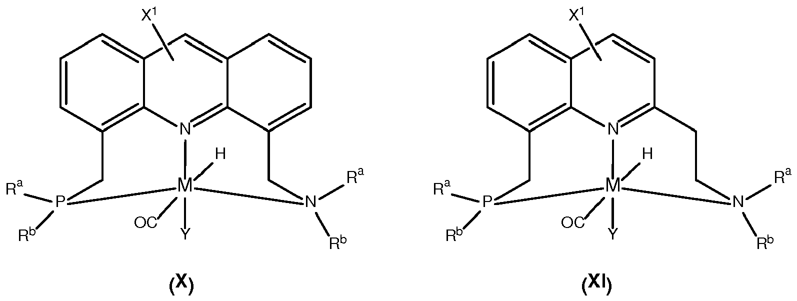 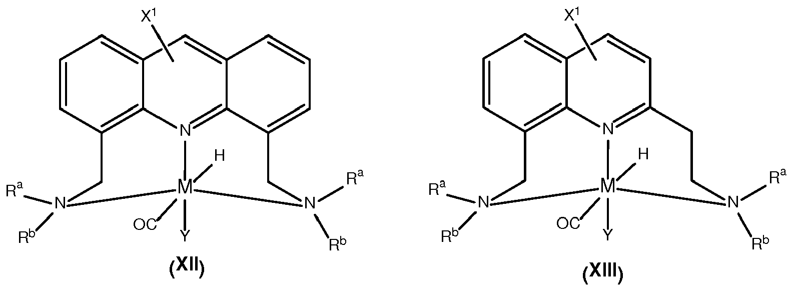 and X¹, R^{a}, R^{b} and Y are as defined in claim 1.

5. The process according to claim 1, wherein the complex catalyst is a catalyst of the formula (XIVa) :

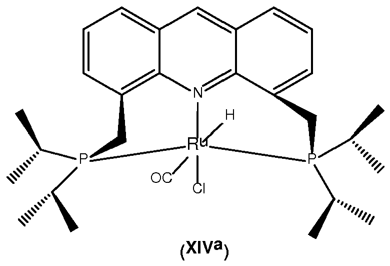

6. The process according to claim 1, wherein the complex catalyst is a catalyst of the formula (XIVb) :

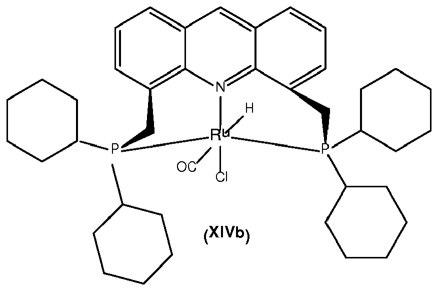

7. The process according to claim 1, wherein the complex catalyst is a catalyst of the formula (XV): 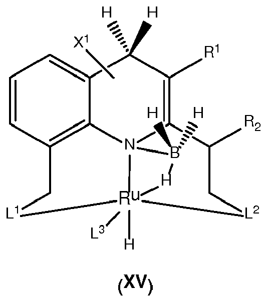 where
L¹ and L² are each, independently of one another, PR^{a}R^{b}, NR^{a}R^{b}, sulfide, SH, S(=O)R, C₅-C₁₀-heteroaryl comprising at least one heteroatom selected from among N, 0 and S, AsR^{a}R^{b}, SbR^{a}R^{b} or N-heterocyclic carbenes of the formula (II) or (III) : 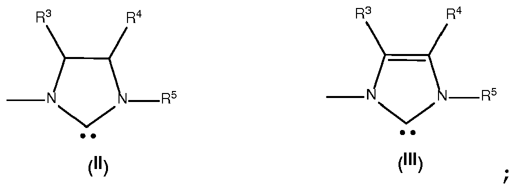
L³ is a monodentate two-electron donor selected from the group consisting of CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophene, tetrahydrothiophene and N-heterocyclic carbenes of the formula (II) or (III);
R¹ and R² are both hydrogen or together with the carbon atoms to which they are bound form a phenyl ring which together with the quinolinyl unit of the formula (I) forms an acridinyl unit;
R, R^{a}, R^{b}, R^{c}, R³, R⁴ and R⁵ are each, independently of one another, unsubstituted or at least monosubstituted C₁-C₁₀-alkyl, C₃-C₁₀-cycloalkyl, C₃-C₁₀-heterocyclyl comprising at least one heteroatom selected from among N, 0 and S, C₅-C₁₀-aryl or C₅-C₁₀-heteroaryl comprising at least one heteroatom selected from among N, O and S,
where the substituents are selected from the group consisting of: F, Cl, Br, OH, CN, NH₂ and C₁-C₁₀-alkyl;
Y is a monoanionic ligand selected from the group consisting of H, F, Cl, Br, I, OCOR, OCOCF₃, OSO₂R, OSO₂CF₃, CN, OH, OR and N(R)₂ or uncharged molecules selected from the group consisting of NH₃, N(R)₃ and R₂NSO₂R;
X¹ represents one, two, three, four, five, six or seven substituents on one or more atoms of the acridinyl unit or one, two, three, four or five substituents on one or more atoms of the quinolinyl unit,
where the radicals X¹ are selected independently from the group consisting of hydrogen, F, Cl, Br, I, OH, NH₂, NO₂,-NC(O)R, C(O)NR₂, -OC(O)R, -C(O)OR, CN and borane derivatives which can be obtained from the catalyst of the formula I by reaction with NaBH₄ and unsubstituted or at least monosubstituted C₁-C₁₀-alkoxy, C₁-C₁₀-alkyl, C₃-C₁₀-cycloalkyl, C₃-C₁₀-heterocyclyl comprising at least one heteroatom selected from among N, 0 and S, C₅-C₁₀-aryl and C₅-C₁₀-heteroaryl comprising at least one heteroatom selected from among N, 0 and S,
where the substituents are selected from the group consisting of: F, Cl, Br, OH, CN, NH₂ and C₁-C₁₀-alkyl;
and
M is iron, cobalt, nickel, ruthenium, rhodium, palladium, osmium, iridium or platinum.

8. The process according to either claim 1 or 7, wherein the complex catalyst is a catalyst of the formula (XVIa) :

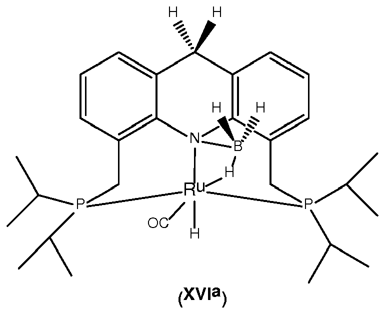

9. The process according to any of claims 1 to 4, wherein Y in the complex catalyst is selected from among H, F, Cl and Br.

10. The process according to any of claims 1 to 9, wherein L³ in the complex catalyst is CO.

11. The process according to any of claims 1 to 10, wherein (-X) is selected from among functional groups of the formulae (-CH₂-OH) and (-CH₂-NH₂).

12. The process according to any of claims 1 to 11, wherein diethylene glycol is used as the diol.

13. The process according to any of claims 1 to 11, wherein a diol selected from the group consisting of ethylene glycol, diethanolamine, polytetrahydrofuran and 1,4-butanediol is used.

14. The use of a complex catalyst comprising at least one element selected from groups 8, 9 and 10 of the Periodic Table and also at least one phosphorus donor ligand for the homogeneously catalyzed preparation of primary amines which have at least one functional group of the formula (-CH₂-NH₂) and at least one further primary amino group by alcohol amination of starting materials having at least one functional group of the formula (-CH₂-OH) and at least one further functional group (-X), where (-X) is selected from among hydroxyl groups and amino groups, by means of ammonia.

## Revendications

1. Procédé de fabrication d'amines primaires, qui comprennent au moins un groupe fonctionnel de formule (-CH₂-NH₂) et au moins un groupe amino primaire supplémentaire, par amination d'alcool de réactifs qui comprennent au moins un groupe fonctionnel de formule (-CH₂-OH) et au moins un groupe fonctionnel supplémentaire (-X), (-X) étant choisi parmi des groupes hydroxyle et des groupes amino primaires, avec de l'ammoniac avec clivage d'eau, dans lequel la réaction est réalisée sous catalyse homogène en présence d'au moins un catalyseur complexe contenant au moins un élément choisi dans les groupes 8, 9 et 10 du tableau périodique, ainsi qu'au moins un ligand donneur,
dans lequel le catalyseur complexe est un catalyseur de formule I : 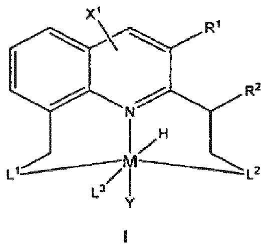 dans laquelle
L¹ et L² signifient indépendamment l'un de l'autre PR^{a}R^{b}, NR^{a}R^{b}, sulfure, SH, S(=O)R, hétéroaryle en C₅-C₁₀ contenant au moins un hétéroatome choisi parmi N, 0 et S, AsR^{a}R^{b}, SbR^{a}R^{b} ou des carbènes N-hétérocycliques de formule II ou III : 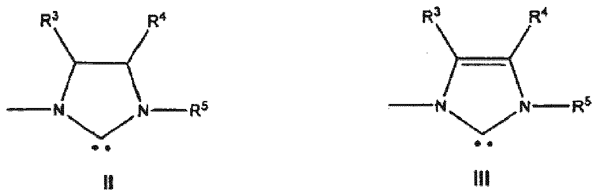
L³ signifie un donneur de deux électrons monodentate choisi dans le groupe constitué par CO, PR^{a}R^{b}R^{c}, NO⁺, AsR^{a}R^{b}R^{c}, SbR^{a}R^{b}R^{c}, SR^{a}R^{b}, RCN, RNC, N₂, PF₃, CS, pyridine, thiophène, tétrahydrothiophène et des carbènes N-hétérocycliques de formule II ou III ;
R¹ et R² signifient tous les deux hydrogène ou forment conjointement avec les atomes de carbone auxquels ils sont reliés un cycle phényle, qui forme conjointement avec l'unité quinolinyle de la formule I une unité acridinyle ;
R, R^{a}, R^{b}, R^{c}, R³, R⁴ et R⁵ signifient indépendamment les uns des autres alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y signifie un ligand monoanionique choisi dans le groupe constitué par H, F, Cl, Br, I, OCOR, OCOCF₃. OSO₂R, OSO₂CF₃, CN, OH, OR et N(R)₂ ou des molécules neutres choisies dans le groupe constitué par NH₃, N(R)₃ et R₂NSO₂R ;
X¹ signifie un, deux, trois, quatre, cinq, six ou sept substituants sur un ou plusieurs atomes de l'unité acridinyle ou un, deux, trois, quatre ou cinq substituants sur un ou plusieurs atomes de l'unité quinolinyle,
les X¹ étant choisis indépendamment les uns des autres dans le groupe constitué par hydrogène, F, Cl, Br, I, OH, NH₂, NO₂, -NC(O)R, C(O)NR₂, -OC(O)R, -C(O)OR, CN et les dérivés de borane, qui peuvent être obtenus à partir du catalyseur de formule I par réaction avec NaBH₄, et alcoxy en C₁-C₁₀, alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ et hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
et
M signifie fer, cobalt, nickel, ruthénium, rhodium, palladium, osmium, iridium ou platine.

2. Procédé selon la revendication 1, dans lequel R¹ et R² signifient tous les deux hydrogène et le catalyseur complexe est un catalyseur de formule (IV) : 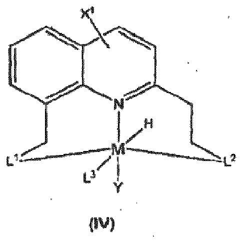 et X¹, L¹, L², L³ et Y ont les significations indiquées dans la revendication 1.

3. Procédé selon la revendication 1, dans lequel R¹ et R² forment conjointement avec les atomes de carbone auxquels ils sont reliés un cycle phényle, qui forme conjointement avec l'unité quinolinyle de la formule I une unité acridinyle, et le catalyseur complexe est un catalyseur de formule (V) : 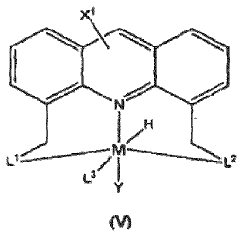 dans laquelle X¹, L¹, L², L³ et Y ont les significations indiquées dans la revendication 1.

4. Procédé selon la revendication 1, dans lequel le catalyseur complexe est choisi dans le groupe des catalyseurs de formule (VI), (VII), (VIII), (IX), (X), (XI), (XII) et (XIII) : 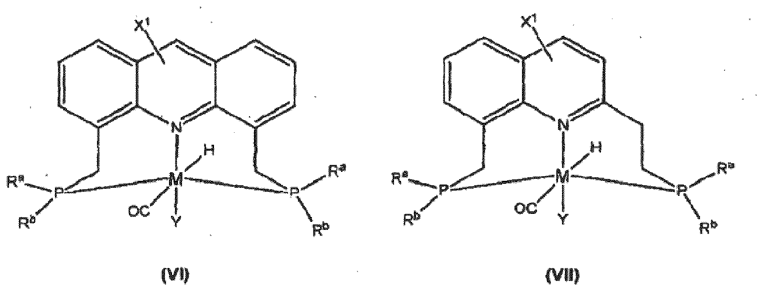 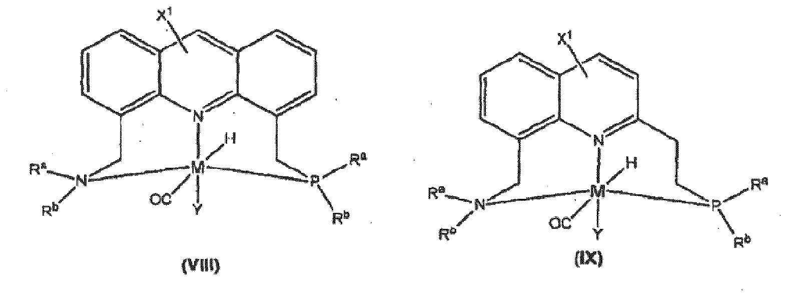 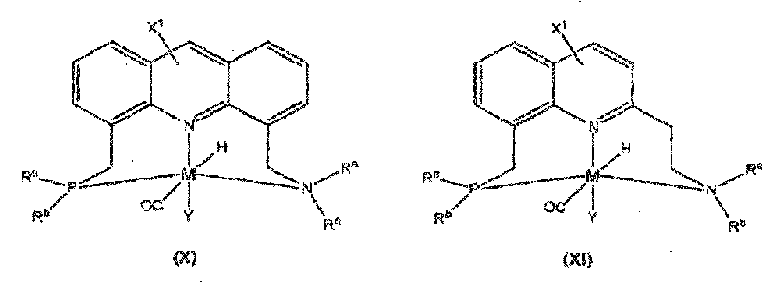 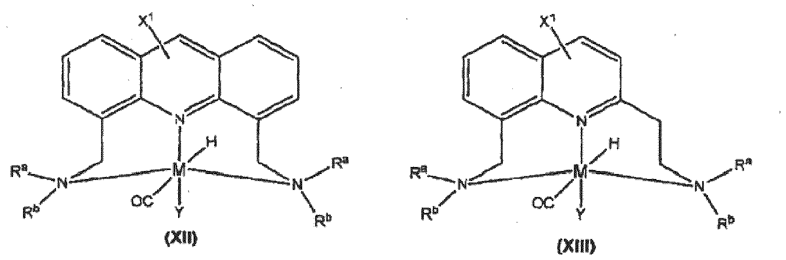 et X¹, R^{a}, R^{b} et Y ont les significations indiquées dans la revendication 1.

5. Procédé selon la revendication 1, dans lequel le catalyseur complexe est un catalyseur de formule (XIVa) :

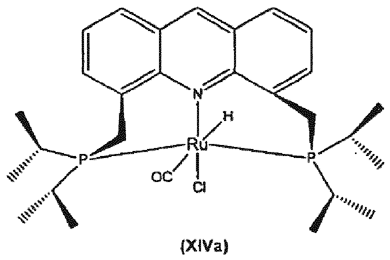

6. Procédé selon la revendication 1, dans lequel le catalyseur complexe est un catalyseur de formule (XIVb) :

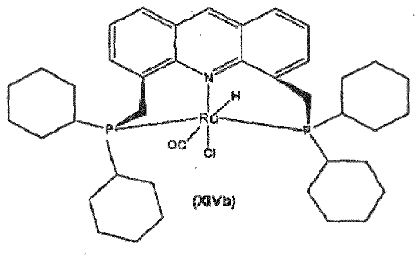

7. Procédé selon la revendication 1, dans lequel le catalyseur complexe est un catalyseur de formule (XV) : 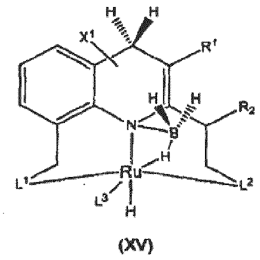 dans laquelle
L¹ et L² signifient indépendamment l'un de l'autre PR^{a}R^{b}, NR^{a}R^{b}, sulfure, SH, S(=O)R, hétéroaryle en C₅-C₁₀ contenant au moins un hétéroatome choisi parmi N, 0 et S, AsR^{a}R^{b}, SbR^{a}R^{b} ou des carbènes N-hétérocycliques de formule (II) ou (III) : 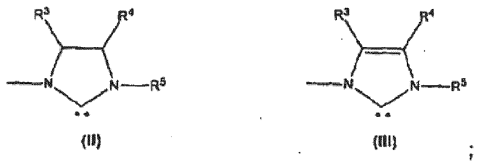
L³ signifie un donneur de deux électrons monodentate choisi dans le groupe constitué par CO, PR^{a}R^{b}R^{c}, NO⁺, AsR^{a}R^{b}R^{c}, SbR³R^{a}R^{b}R^{c}, SR^{a}R^{b}, RCN, RNC, N₂, PF₃, CS, pyridine, thiophène, tétrahydrothiophène et des carbènes N-hétérocycliques de formule (II) ou (III) ;
R¹ et R² signifient tous les deux hydrogène ou forment conjointement avec les atomes de carbone auxquels ils sont reliés un cycle phényle, qui forme conjointement avec l'unité quinolinyle de la formule (I) une unité acridinyle ;
R, R^{a}, R^{b}, R^{c}, R³, R⁴ et R⁵ signifient indépendamment les uns des autres alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ ou hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
Y signifie un ligand monoanionique choisi dans le groupe constitué par H, F, Cl, Br, I, OCOR, OCOCF₃. OSO₂R, OSO₂CF₃, CN, OH, OR et N(R)₂ ou des molécules neutres choisies dans le groupe constitué par NH₃, N(R)₃ et R₂NSO₂R ;
X¹ signifie un, deux, trois, quatre, cinq, six ou sept substituants sur un ou plusieurs atomes de l'unité acridinyle ou un, deux, trois, quatre ou cinq substituants sur un ou plusieurs atomes de l'unité quinolinyle,
les X¹ étant choisis indépendamment les uns des autres dans le groupe constitué par hydrogène, F, Cl, Br, I, OH, NH₂, NO₂, -NC(O)R, C(O)NR₂, -OC(O)R, -C(O)OR, CN et les dérivés de borane, qui peuvent être obtenus à partir du catalyseur de formule I par réaction avec NaBH₄, et alcoxy en C₁-C₁₀, alkyle en C₁-C₁₀, cycloalkyle en C₃-C₁₀, hétérocyclyle en C₃-C₁₀ contenant au moins un hétéroatome choisi parmi N, 0 et S, aryle en C₅-C₁₀ et hétéroaryle en C₅-C₁₀ contenant au moins un hétéroatome choisi parmi N, 0 et S, non substitué ou au moins monosubstitué,
les substituants étant choisis dans le groupe constitué par : F, Cl, Br, OH, CN, NH₂ et alkyle en C₁-C₁₀ ;
et
M signifie fer, cobalt, nickel, ruthénium, rhodium, palladium, osmium, iridium ou platine.

8. Procédé selon l'une quelconque des revendications 1 ou 7, dans lequel le catalyseur complexe est un catalyseur de formule (XVIa) :

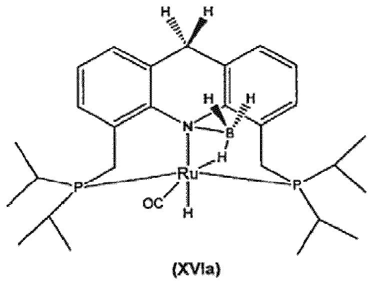

9. Procédé selon l'une quelconque des revendications 1 à 4, dans lequel Y dans le catalyseur complexe est choisi parmi H, F, Cl et Br.

10. Procédé selon l'une quelconque des revendications 1 à 9, dans lequel L³ dans le catalyseur complexe représente CO.

11. Procédé selon l'une quelconque des revendications 1 à 10, dans lequel (-X) est choisi parmi des groupes fonctionnels de formules (-CH₂-OH) et (-CH₂-NH₂) .

12. Procédé selon l'une quelconque des revendications 1 à 11, dans lequel on utilise du diéthylène glycol en tant que diol.

13. Procédé selon l'une quelconque des revendications 1 à 11, dans lequel on utilise un diol choisi dans le groupe constitué par éthylène glycol, diéthanolamine, polytétrahydrofurane et 1,4-butanediol.

14. Utilisation d'un catalyseur complexe qui contient au moins un élément choisi dans les groupes 8, 9 et 10 du tableau périodique, ainsi qu'au moins un ligand donneur de phosphore, pour la fabrication sous catalyse homogène d'amines primaires, qui comprennent au moins un groupe fonctionnel de formule (-CH₂-NH₂) et au moins un groupe amino primaire supplémentaire, par amination d'alcool de réactifs qui comprennent au moins un groupe fonctionnel de formule (-CH₂-OH) et au moins un groupe fonctionnel supplémentaire (-X), (-X) étant choisi parmi des groupes hydroxyle et des groupes amino primaires, avec de l'ammoniac.
